# EUROPÄISCHE PATENTSCHRIFT

(11) **EP 0 944 631 B1**
(45) Veröffentlichungstag und Bekanntmachung des Hinweises auf die Patenterteilung: **18.02.2004**
(21) Anmeldenummer: 97951204.3
(22) Anmeldetag: 14.11.1997
(51) Int. Cl.: C07D 487/04, C07D 471/04, A61K 31/435, A61K 31/495, A61K 31/415

(54) **NEUE SUBSTITUIERTE PYRAZOLDERIVATE ZUR BEHANDLUNG VON HERZKREISLAUFERKRANKUNGEN**
NOVEL SUBSTITUTED PYRAZOLE DERIVATIVES FOR THE TREATMENT OF CARDIOCIRCULATORY DISEASES
NOUVEAUX DERIVES DE PYRAZOLE SUBSTITUES POUR LE TRAITEMENT DE MALADIES CARDIO-VASCULAIRES

(30) Priorität: 26.11.1996 DE 19649460
(43) Veröffentlichungstag der Anmeldung: 29.09.1999
(73) Patentinhaber: Bayer HealthCare AG, 51368 Leverkusen (DE)
(72) Erfinder: STRAUB, Alexander, D-42113 Wuppertal (DE); ROBYR, Chantal, D-45470 Mülheim (DE); JAETSCH, Thomas, D-50668 Köln (DE); FEURER, Achim, D-51519 Odenthal (DE); KAST, Raimund, D-42389 Wuppertal (DE); STASCH, Johannes-Peter, D-42651 Solingen (DE); PERZBORN, Elisabeth, D-42327 Wuppertal (DE); HÜTTER, Joachim, D-42349 Wuppertal (DE); DEMBOWSKY, Klaus, D-42115 Wuppertal (DE)
(86) Internationale Anmeldenummer: PCT/EP1997/006366
(87) Internationale Veröffentlichungsnummer: WO 1998/023619

(56) Entgegenhaltungen:
- EP-A- 0 254 241
- EP-A- 0 417 449
- EP-A- 0 641 564
- EP-A- 0 667 345
- CHEMICAL ABSTRACTS, vol. 125, no. 3, 15.Juli 1996 Columbus, Ohio, US; abstract no. 33633m, GUO,S. ET AL.: "Preparation of condensed 1-benzyl-3-aryl-pyrazole derivatives as blood platelet aggregation inhibitors" Seite 903; XP002061264 & CN 1 112 926 A (GUO,S. ET AL.) 6.Dezember 1995

## Beschreibung

Die vorliegende Erfindung betrifft neue substituierte Pyrazol-Derivate, Verfahren zu ihrer Herstellung und ihre. Verwendung als Arzneimittel, insbesondere als Arzneimittel zur Behandlung von Herz-Kreislauf-Erkrankungen.

Es ist bereits bekannt, daß 1-Benzyl-3-(substituierte heteroaryl)-kondensierte Pyrazol-Derivate die Thrombozytenaggregation inhibieren (vgl. EP 667 345 A1 sowie CN-A-1 112 926 [ C.A. 125: 33633m (1996) ] ).

Weiterhin sind in EP-A-0 417 449, EP-A-0 254241 und Chin. Pharm. J. 47, 407 (1995) unterschiedliche Pyrazolderivate beschrieben, die ebenfalls die Thrombozytenaggregation inhibieren und somit zur Behandlung von Thrombosen geeignet sind.

EP-A-0 641 564 offenbart Pyrazolderivate, die sich zur Behandlung von Thrombozytopenie (Thrombopenie) eignen.

Die vorliegende Erfindung betrifft neue substituierte Pyrazolderivate der allgemeinen Formel (I), 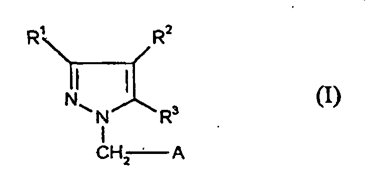 in welcher die Substituenten R¹, R², R³ und A wie in Anspruch 1 definiert sind.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexyiamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen.

Umfasst sind erfindungsgemäße Verbindungen der allgemeinen Formel (I),
in welcher
- R¹: für Imidazolyl, Pyridyl, Pyrimidyl oder Oxazolyl steht, die gegebenenfalls bis zu 3-fach gleich oder verschieden durch Formyl, Fluor, Chlor, Amino, Mercaptyl, Cyano, geradkettiges oder verzweigtes Acyl, Alkylthio, Alkoxy oder Alkoxycarbonyl mit jeweils bis zu 4 Kohlenstoffatomen oder geradkettiges oder verzweigtes Alkyl mit bis zu 4 Kohlenstoffatomen substituiert sind, das seinerseits durch Hydroxy, Carboxyl, Amino, Azido, geradkettiges oder verzweigtes Acyl, Alkoxy, Alkoxycarbonyl oder Acylamino mit jeweils bis zu 3 Kohlenstoffatomen substituiert sein kann,
und/oder durch einen Rest der Formel 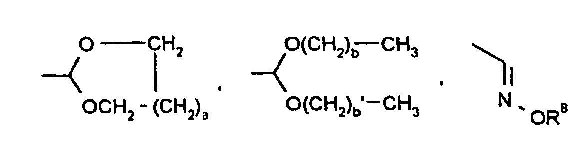 oder
-S(O)_{c}-NR⁹R¹⁰ substituiert sind,
worin
a, b b' und gleich oder verschieden sind und eine Zahl 0, 1 oder 2 bedeuten,
R⁸ Wasserstoff oder Methyl bedeutet,
c eine Zahl 1 oder 2 bedeutet und R⁹ und R¹⁰ gleich oder verschieden sind und Wasserstoff oder geradkettiges oder verzweigtes Alkyl mit bis zu 9 Kohlenstoffatomen bedeuten, das gegebenenfalls durch Phenyl oder Naphthyl substituiert sein können, oder
Phenyl oder Naphthyl bedeuten, die gegebenenfalls durch Fluor oder Chlor substituiert sind, oder
Cyclopropyl oder Cycloheptyl bedeuten, oder
R⁹ und R¹⁰ gemeinsam mit dem Stickstoffatom einen Morpholinring oder einen Rest der Formel 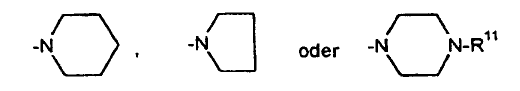 bilden, worin
R¹¹ Wasserstoff, Methyl oder einen Rest der Formel 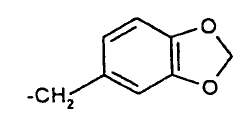 bedeutet oder Benzyl oder Phenyl
bedeutet, wobei die Ringsysteme gegebenenfalls durch Chlor substituiert sind,
- R² und R³: unter Einbezug der Doppelbindung einen Pyridyl-, Pyrazinyl- Pyrimidinyl- oder Pyridazinylring bilden, die gegebenenfalls bis zu 3-fach gleich oder verschieden durch Formyl, Mercaptyl, Carboxyl, Hydroxy, geradkettiges oder verzweigtes Acyl, Alkoxy, Alkylthio oder Alkoxycarbonyl mit jeweils bis zu 4 Kohlenstoffatomen, Nitro, Cyano, Fluor, Chlor oder geradkettiges oder verzweigtes Alkyl oder Alkoxy mit jeweils bis zu 3 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 3 Kohlenstoffatomen substituiert sein kann,
und/oder die heterocyclischen Ringe gegebenenfalls durch Amino, N,N-- Dimethylamino oder durch einen Rest der Formel -NH-CHO oder - N=CH-N(CH₃)₂ substituiert sind und/oder durch Phenyl substituiert sind,
das seinerseits durch einen Rest der Formel -O(CH₂)₂-CH₃ substituiert sein kann,
- A: für Tetrahydropyranyl, Phenyl, Pyrimidyl, Thienyl oder Pyridyl steht, die gegebenenfalls bis zu 2-fach gleich oder verschieden durch Formyl, Carboxyl, geradkettiges oder verzweigtes Acyl, Alkylthio, Alkyloxyacyl, Alkoxy oder Alkoxycarbonyl mit jeweils bis zu 3 Kohlenstoffatomen, Fluor, Chlor, Brom, Nitro, Cyano, Trifluormethyl, oder geradkettiges oder verzweigtes Alkyl mit bis zu 3 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 3 Kohlenstoffatomen substituiert sein kann,
und deren isomere Formen und Salze.

Ganz besonders bevorzugt sind erfindungsgemäße Verbindungen der allgemeinen Formel (I), in welcher
- A: für Phenyl, Pyrimidyl oder für durch Fluor substituiertes Phenyl oder Pyrimidyl steht
und deren isomere Formen und Salze.

Außerdem wurden Verfahren zur Herstellung der erfindungsgemäßen Verbindungen der allgemeinen Formel (I) gefunden, dadurch gekennzeichnet, daß man in Abhängigkeit der verschiedenen Bedeutungen der oben unter R² und R³ aufgeführten Heterocyclen
[A] Verbindungen der allgemeinen Formel (II)

   R¹-D (II)

   in welcher
   - R¹: die oben angegebene Bedeutung hat,
   und
   - D: für Reste der Formel 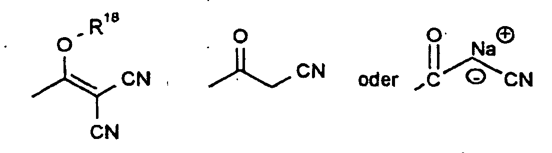 steht,
   in welchen
   R¹⁸ für C₁-C₄-Alkyl steht, durch Umsetzung mit Verbindungen der allgemeinen Formel (III).

   A-CH₂-NH-NH₂ (III)

   in welcher
   A die oben angegebene Bedeutung hat in inerten Lösemitteln, gegebenenfalls in Anwesenheit einer Base, in die Verbindungen der allgemeinen Formel (IV) oder (IVa) 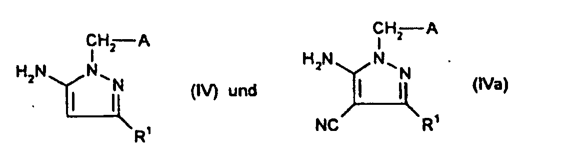 in welcher
   A und R¹ die oben angegebene Bedeutung haben,
   überführt,
   und im Fall der Verbindungen der allgemeinen Formel (IVa) anschließend mit Carbonsäuren, Nitrilen, Formamiden oder Guanidiumsalzen cyclisiert,
   und im Fall der Verbindungen der allgemeinen Formel (IV) mit 1,3Dicarbonyl-Derivaten, deren Salze, Tautomeren, Enolether oder Enaminen, in Anwesenheit von Säuren und gegebenenfalls unter Mikrowellen cyclisiert,
   oder
[B] im Fall, daß R² und R³ gemeinsam einen Pyrazinring bilden, Verbindungen der allgemeinen Formel (IV) zunächst durch Nitrosierung in die Verbindungen der allgemeinen Formel (V) 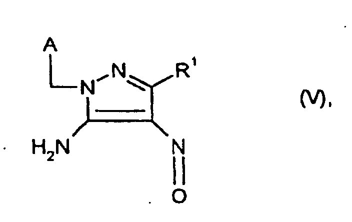 in welcher
   A und R¹ die oben angegebene Bedeutung haben,
   überführt,
   in einem zweiten Schritt durch eine Reduktion die Verbindungen der allgemeinen Formel (VI) 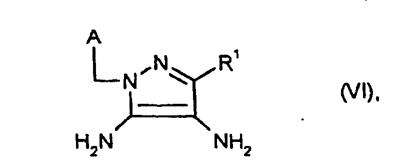 in welcher
   A und R¹ die oben angegebene Bedeutung haben,
   herstellt,
   und abschließend mit 1,2-Dicarbonylverbindungen, vorzugsweise wäßriger Glyoxallösung cyclisiert,
   oder
[C] Verbindungen der allgemeinen Formel (VII) 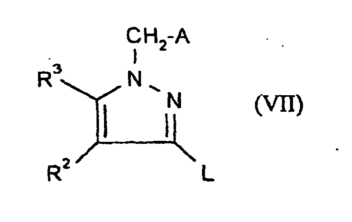 in welcher
   A¹, R² und R³ die oben angegebene Bedeutung haben,
   und
   - L: für einen Rest der Formel -SnR¹⁹R²⁰R²¹, ZnR²², Iod, Brom oder Triflat steht,
   worin
   R¹⁹, R²⁰ und R²¹ gleich oder verschieden sind und geradkettiges oder verzweigtes Alkyl mit bis zu 4 Kohlenstoffatomen bedeuten,
   und
   R²² Halogen bedeutet,
   mit Verbindungen der allgemeinen Formel (VIII)

   R¹-T (VIII)

   in welcher
   - R¹: die oben angegebene Bedeutung hat
   und
   im Fall L = SnR¹⁹R²⁰R²¹ oder ZnR²²
   - T: für Triflat oder für Halogen, vorzugsweise für Brom steht,
   und
   im Fall L = Jod, Brom oder Triflat
   - T: für einen Rest der Formel SnR^{19'}R^{20'}R^{21'}, ZnR^{22'} oder BR^{23'}R^{24'} steht,
   worin
   R^{19'}, R^{20'}, R^{21'} und R^{22'} die oben angebene Bedeutung von R¹⁹, R²⁰, R²¹ und R²² haben und mit dieser gleich oder verschieden sind,
   R^{23'} und R^{24'} gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
   in einer palladiumkatalysierten Reaktion in inerten Lösemitteln umsetzt, gegebenenfalls in Gegenwart einer Base,
[D] im Fall R¹ = 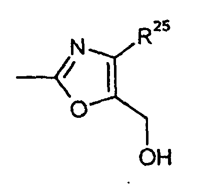
worin
- R²⁵: (C₁-C₄)-Alkyl bedeutet,
Verbindungen der allgemeinen Formel (IX) 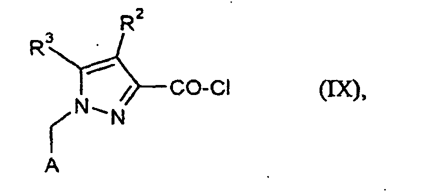 in welcher
A, R² und R³ die oben angegebene Bedeutung haben,
entweder direkt durch Umsetzung mit der Verbindung der Formel (X) 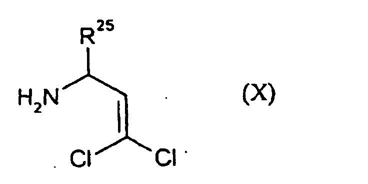 in welcher
- R²⁵: die oben angegebene Bedeutung hat,
in dem System NaOCO-CH₃/N-Methylpyrrolidin
in die Verbindungen der allgemeinen Formel (Ia) 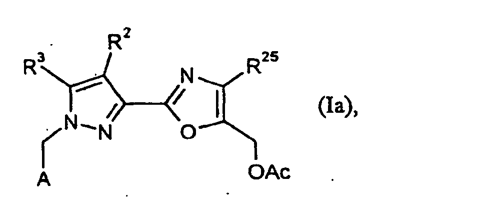 in welcher
R², R³ und A und R²⁵ die oben angegebene Bedeutung haben,
überführt,
und anschließend durch Einwirkung von Kaliumhydroxid in Methanol die Acetylgruppe abspaltet,
oder
zunächst durch Umsetzung der Verbindungen der allgemeinen Formel (IX) mit der Verbindung der Formel (X) die Verbindungen der allgemeinen Formel (XI) 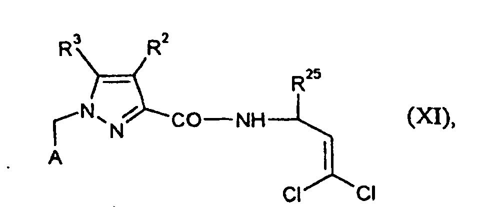 in welcher
R², R³, A und R²⁵ die oben angegebene Bedeutung haben,
herstellt,
und in einem weiteren Schritt durch Einwirkung von Kaliumhydroxid die Hydroxymethylverbindungen herstellt,
und im Fall der Gruppen -S(O)_{c}NR⁹R¹⁰ und -S(O)_{c'}NR^{9'}R^{10'} ausgehend von den unsubstituierten Verbindungen der allgemeinen Formel (I) zunächst mit Thionylchlorid und in einem zweiten Schritt mit den entsprechenden Aminen umsetzt
und gegebenenfalls die unter R¹, R², R³ und/oder A aufgeführten Substituenten nach üblichen Methoden, vorzugsweise durch Chlorierung, katalytische Hydrierung, Reduktion, Oxidation, Abspaltung von Schutzgruppen und/oder nucleophiler Substitution variiert oder einführt.

Die unter R² und R³ aufgeführten Heterocyclen können auch durch Umsetzung der entsprechend substituierten Verbindungen der allgemeinen Formel (II) nach anderen bekannten heterocyclischen Synthesen eingeführt werden.

Das erfindungsgemäße Verfahren kann durch folgendes Formelschema beispielhaft erläutert werden:

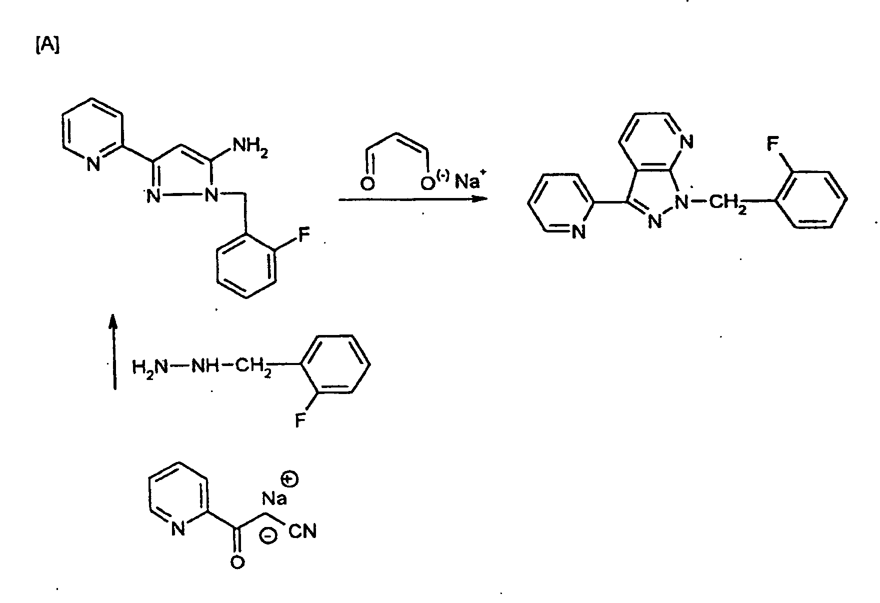

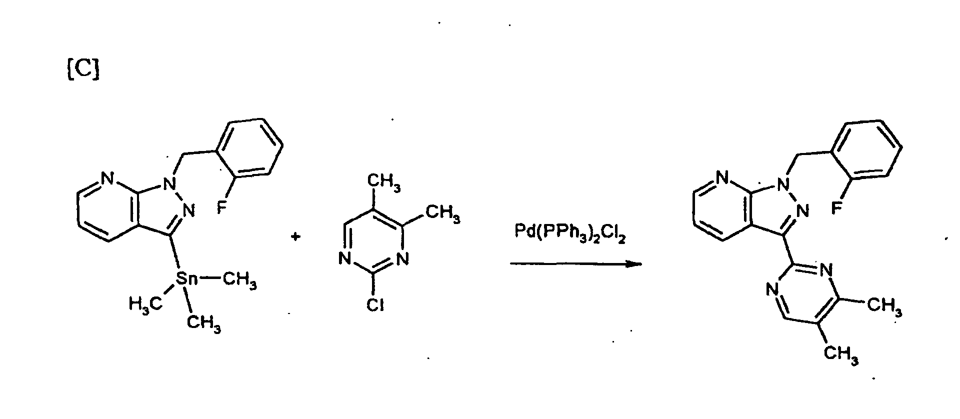

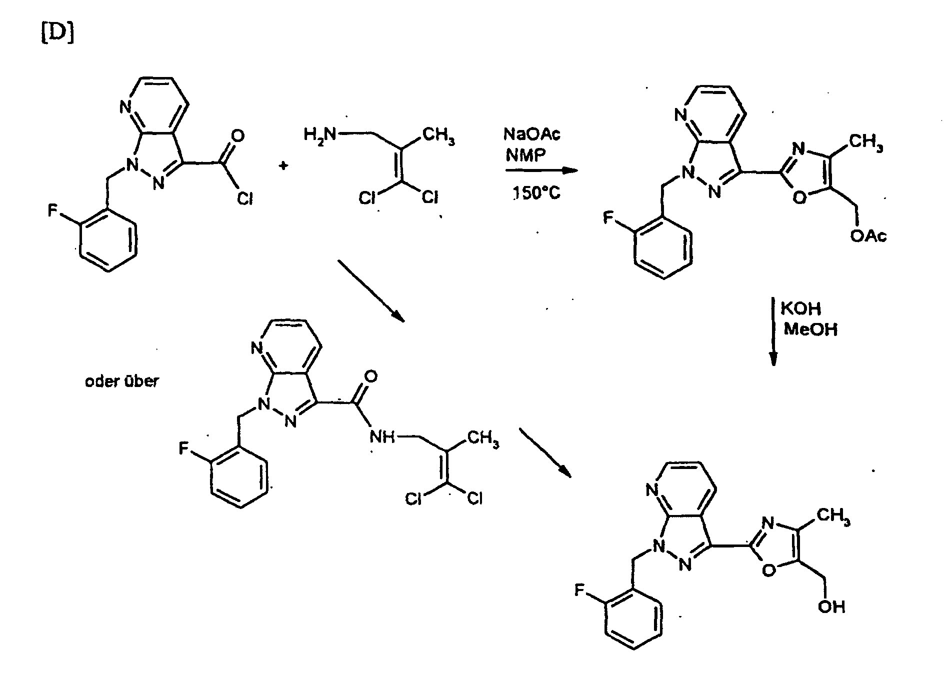

Als Lösemittel für die einzelnen Schritte der Verfahren eignen sich hierbei inerte. organische Lösemittel, die sich unter den Reaktionsbedingungen nicht verändern. Hierzu gehören Ether, wie Diethylether oder Tetrahydrofuran, DME, Dioxan, Alkohole wie Methanol und Ethanol, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol,Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Tetrahydrofuran, Dimethylformamid, Toluol, Dioxan oder Dimethoxyethan.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkalioder Erdalkalialkoholate wie Natrium- oder Kaliummethanolat, Natrium- oder Kaliumethanolat oder Kalium-tert.butylat, oder organische Amine (Trialkyl(C₁-C₆)-amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat, Triethylamin und Natriumhydrid.

Die Base wird in einer Menge von 1 mol bis 5 mol, bevorzugt von 1 mol bis 3 mol, bezogen auf 1 mol der Verbindung der allgemeinen Formel (II) eingesetzt.

Die Umsetzung wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Als Säuren für die Cyclisierung eignen sich im allgemeinen Protonensäuren. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure.

Die katalytische Hydrierung kann im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Die Chlorierung erfolgt im allgemeinen mit den üblichen Chlorierungsmitteln wie beispielsweise PCl₃, PCl₅, POCl₃ oder elementarem Chlor. Bevorzugt ist im Rahmen der Erfindung POCl₃.

Im Fall, daß die Reste der Formeln -S(O)_{c}NR⁹R¹⁰ und -S(O)_{c'}NR^{9'}R^{10'} vorliegen, werden die entsprechenden unsubstituierten Verbindungen zunächst mit Thionylchlorid umgesetzt. In einem weiteren Schritt erfolgt die Umsetzung mit den Aminen in einem der oben aufgeführten Ether, vorzugsweise Dioxan. Im Fall c = 2 wird anschließend eine Oxidation nach üblichen Methoden durchgeführt. Die Umsetzungen erfolgen in einem Temperaturbereich von 0°C bis 70°C und Normaldruck.

Die nucleophilen Substitutionen und Vilsmeierreaktionen werden nach üblichen, publizierten Methoden durchgeführt.

Die Reduktionen werden im allgemeinen mit Reduktionsmitteln, bevorzugt mit solchen, die für die Reduktion von Carbonyl zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im allgemeinen in einem Temperaturbereich von -78°C bis +50°C, bevorzugt von -78°C bis 0°C im Falle des DIBAH, 0°C bis Raumtemperatur im Falle des NaBH_{4.}

Die Reduktion verläuft im allgemeinen bei Normaldruck, es ist aber auch möglich bei erhöhtem oder erniedrigtem Druck zu arbeiten.

Die Verbindungen der allgemeinen Formeln (II) und (III) sind an sich bekannt oder nach üblichen Methoden herstellbar (vgl. hierzu: J. Hromatha et al., Monatsh. Chem. 1976, 107, 233).

Die Verbindungen der allgemeinen Formeln (IV), (IVa), (V) und (VI) sind teilweise bekannt und können wie oben beschrieben hergestellt werden.

Als Lösemittel für das Verfahren [C] eignen sich hierbei inerte organische Lösemittel, die sich unter den Reaktionsbedingungen nicht verändern. Hierzu gehören Ether, wie Diethylether oder Tetrahydrofuran, DME, Dioxan, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Tetrahydrofuran, Dimethylformamid, Toluol, Dioxan oder Dimethoxyethan.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Als Palladiumverbindungen im Rahmen der vorliegenden Erfidung eignen sich im allgemeinen PdCl₂(P(C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂) oder Pd(P(C₆H₅)₃)₄. Bevorzugt ist Pd(P(C₆H₅)₃)₄.

Die Verbindungen der allgemeinen Formel (VIII) sind an sich bekannt oder nach üblichen Methoden herstellbar.

Die Verbindungen der allgemeinen Formel (VII) sind teilweise bekannt oder im Fall der Stannyle neu und können dann beispielsweise hergestellt werden, indem man die Verbindungen der allgemeinen Formel (XII) 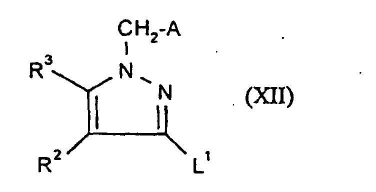 in welcher
R², R³ und A die oben angegebene Bedeutung haben,
- L¹: für Triflat oder Halogen, vorzugsweise für Iod steht,
mit Verbindungen der allgemeinen Formel (XIII)

(SnR¹⁹R²⁰R²¹)₂ (XIII)

in welcher
R¹⁹, R²⁰, R²¹ die oben angegebene Bedeutung haben
wie oben beschrieben palladiumkatalysiert umsetzt.

Die Verbindungen der allgemeinen Formeln (XII) und (XIII) sind an sich bekannt oder nach üblichen Methoden herstellbar.

Das erfindungsgemäße Verfahren [D] erfolgt mit einer der oben aufgeführten Basen, vorzugsweise in N-Methylpyrrolidon, in einem Temperaturbereich von 100°C bis 200°C, vorzugsweise bei 150°C.

Die Verbindungen der allgemeinen Formeln (IX) und (X) sind bekannt oder nach üblichen Methoden herstellbar.

Die Verbindungen der allgemeinen Formeln (Ia) und (XI) sind neu und können wie oben beschrieben hergestellt werden.

Für den Fall, daß typische Schutzgruppen im Rahmen von Derivatisierungsreaktionen eingesetzt werden, erfolgt deren Abspaltung im allgemeinen in einem der oben aufgeführten Alkohole und/oder THF oder Aceton, vorzugsweise Methanol / THF in Anwesenheit von Salzsäure oder Trifluoressigsäure oder Toluolsulfonsäure in einem Temperaturbereich von 0°C bis 70°C, vorzugsweise bei Raumtemperatur und Normaldruck.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I) führen zu einer Gefäßrelaxation/Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen. Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose und Krankheiten des Urogenitalsystems wie beispielsweise Prostatahypertrophie, erektile Dysfunktion und Inkontinenz eingesetzt werden.

Darüber hinaus umfaßt die Erfindung die Kombination der erfindungsgemäßen Verbindungen der allgemeinen Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfaßt die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindungen potenziert und der gewünschte pharmakologische Effekt gesteigert.

Zur Feststellung der kardiovaskulären Wirkungen wurden folgende Untersuchungen durchgeführt: In in vitro-Untersuchungen an Zellen vaskulären Ursprungs wurde der Einfluß auf die Guanylatzyklase-abhängige cGMP-Bildung mit und ohne NO-Donor geprüft. Die antiaggregatorischen Eigenschaften wurden an mit Kollagen stimulierten menschlichen Thrombozyten gezeigt. Die gefäßrelaxierende Wirkung wurde an mit Phenylephrin vorkontrahierten Kaninchenäortenringen bestimmt. Die blutdrucksenkende Wirkung wurde an narkotisierten Ratten untersucht.

### Stimulation der löslichen Guanylatzyklase in primären Endothelzellen

Primäre Endothelzellen wurden aus Schweineaorten durch Behandlung mit Kollagenase-Lsg. isoliert. Anschließend wurden die Zellen in Kulturmedium bei 37°C / 5% CO₂ bis zum Erreichen der Konfluenz kultiviert. Für die Untersuchungen wurden die Zellen passagiert, in 24-Loch Zellkulturplatten ausgesät und bis zum Erreichen der Konfluenz subkultiviert (~ 2 x 10⁵ Zellen / Vertiefung). Zur Stimulation der endothelialen Guanylatzyklase wurde das Kulturmedium abgesaugt und die Zellen einmal mit Ringerlösung gewaschen. Nach Entfernen der Ringerlösung wurden die Zellen in Stimulationspuffer mit oder ohne NO-Donor (Natrium-Nitroprussid, SNP, 1 µM) 10 Minuten bei 37°C / 5% CO₂ inkubiert. Im Anschluß daran wurden die Testsubstanzen (Endkonzentration 1 µM) zu den Zellen pipettiert und weitere 10 Minuten inkubiert. Nach Ende der Inkubationszeit. wurde die Pufferlösung abgesaugt und 4°C kalter Stoppuffer zu den Zellen gegeben. Die Zellen wurden dann 16 Stunden lang bei -20°C lysiert. Anschließend wurden die das intrazelluläre cGMP enthaltenden Überstände abgenommen und die cGMP-Konzentrationen durch das cGMP-SPA-System (Amersham Buchler, Braunschweig) bestimmt.

**Tabelle A**

| Bsp.-Nr. | cGMP-Steigerung (%) |
|---|---|
| 14* | > 1000. |
| 15* | 504 |
| 16 | 652 |
| 17 | > 1000 |
| 32* | 135 |

| | |
|---|---|
| *Referenzbeispiel | |

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10.
Die Kontraktionskraft wird mit Statham UC2-Zellen erfaßt, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt.

Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀). Das Standardapplikationsvolumen beträgt 5 µl, der DMSO-Anteil in der Badlösung entspricht 0,1 %.

**Tabelle B**

| Bsp.-Nr. | Aorta (IC₅₀) µM |
|---|---|
| 14* | 1,8 |
| 15* | 13,0 |
| 16 | 1,7 |

| | |
|---|---|
| * Referenzbeispiel | |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden als Suspension in Tyloselösung mittels Schlundsonde in verschiedenen Dosen oral verabreicht.

**Tabelle C**

| Bsp.-Nr. | Dosis (mg/kg) | Max. Blutdrucksenkung (mmHg) | Zeit (min) |
|---|---|---|---|
| 13* | 10 | -13 | 60 |
| | 30 | -23 | 60 |
| 14* | 10 | -18 | 40 |
| | 30 | -21 | 50 |
| 16 | 10 | -9 | 50 |
| | 30 | -16 | 50 |

| | | | |
|---|---|---|---|
| * Referenzbeispiel | | | |

### Thrombozytenaggregationshemmung in vitro

Zur Bestimmung der thrombozytenaggregationshemmenden Wirkung wurde Blut von gesunden Probanden beiderlei Geschlechts verwendet. Als Antikoagutans wurden einem Teil 3,8%iger wäßriger Natriumzitratlösung 9 Teile Blut zugemischt. Mittels Zentrifugation erhält man aus diesem Blut plättchenreicheres Zitratplasma (PRP) (Jürgens / Beller, Klinische Methoden der Blutgerinnungsanalyse; Thieme Verlag, Stuttgart, 1959).

Für diese Untersuchungen wurden 445 µl PRP und 5 µl der Wirkstofflösung bei 37°C im Wasserbad vorinkubiert. Anschließend wurde die Thrombozytenaggregation nach der turbidometrischen Methode (Born, G.V.R., J. Physiol. (London), 168, 178-195, 1963) im Aggregometer bei 37°C bestimmt. Hierzu wurde die vorinkubierte Probe mit 50 µl Kollagen, einem aggregationsauslösenden Agens, versetzt, und die Veränderung der optischen Dichte erfaßt. Zur quantitativen Auswertung wurde der maximale Aggregationsresponse ermittelt und daraus die prozentuale Hemmung gegenüber der Kontrolle errechnet.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme.

Weiterhin eignen sich diese Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen zur Bekämpfung von Schmerzzuständen eingesetzt werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

### Abkürzungen

| | |
|---|---|
| MeOH = | Methanol |
| E = | Ether |
| EE = | Essigester |
| T = | Toluol |
| Ph = | Phenyl |

Die Zahlen hinter den Lösemittelabkürzungen in den folgenden Tabellen unter der Spalte R_{f} bedeuten Gewichtsteile.

### Ausgangsverbindungen

### Beispiel I A (Referenzbeispiel)

### 5-Amino-1-benzyl-3-(5-hydroxymethyl-2-furyl)-pyrazol

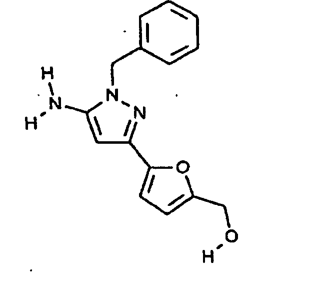

Zu einer Lösung von 79.5 g (408 mmol) Benzylhydrazin-dihydrochlorid in 1.3 l Ethanol gibt man unter Rühren 44.1g (817 mmol) Natriummethanolat. Nach 15 min gibt man 67.4 g (408 mmol) 2-Cyanomethylcarbonyl-5-hydroxymethylfuran hinzu und rührt 3 Stunden unter Rückfluß. Man gibt nach Abkühlen 1 l Wasser hinzu, verdampft den Ethanolanteil im Vakuum und saugt die ausgefallenen Kristalle ab. Nach Waschen mit Wasser und anschließend mit Ether wird über P₂O₅ getrocknet. Man erhält 91 g (83 % d. Th.)Produkt mit einem Smp. von 163°C.

Analog wurden die in der Tabelle 1A aufgeführten Verbindungen hergestellt:

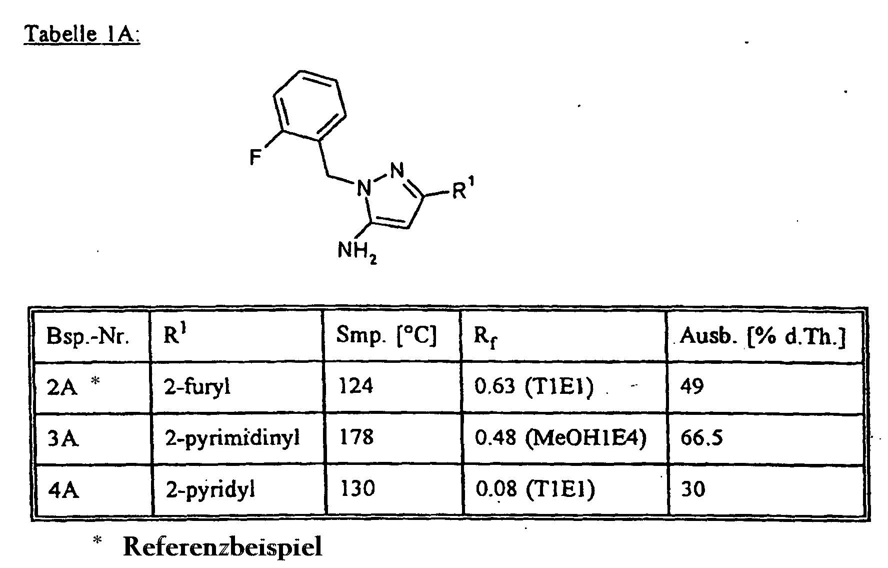

### Beispiel 5A (Referenzbeispiel)

### 5-Amino-1-(2-fluorbenzyl)-3-(5-hydroxymethyl-2-furyl)-4-nitroso-pyrazol

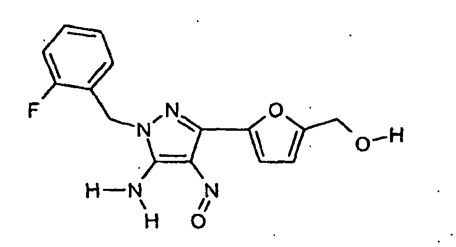

10 g (34,8 mmol) 5-Amino-1-(2-fluorbenzyl)-3-(5-hydroxymethyl-2-furyl)-pyrazol werden in einer Mischung aus 66 ml Ethanol und 26,7 ml 5%iger wäßriger Salzsäure vorgelegt, innerhalb von 5 Minuten mit 26,4 ml einer 15%igen ethanolischen Lösung von Ethylnitrit versetzt und 1 h bei Raumtemperatur gerührt. Die tiefviolette Reaktionslösung wird in wäßrige Kaliumcarbonatlösung gegeben und mit Essigester extrahiert. Nach Verdampfen der organischen Phase im Vakuum erhält man 8 g des Rückstandes, der unmittelbar weiter umgesetzt werden kann. (R_{f} = 0,17, T1E1, SiO₂).

### Beispiel 6A (Referenzbeispiel)

### 4,5-Diamino-1-(2-fluorbenzyl)-3-(5-hydroxymethyl-2-furyl)-pyrazol

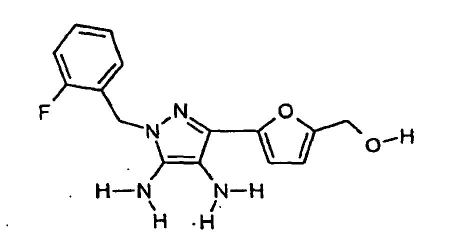

Die Verbindung aus Beispiel 5A (8 g) wird in Ethanol gelöst, mit 0,5 g 5%igem Palladium auf Kohle versetzt und in einer Parr-Apparatur bei 2 bar Wasserstoffdruck 15 Minuten lang hydriert. Man saugt die Lösung über Kieselgur ab und verwendet sie für den nächsten Ansatz (R_{f} = 0,21, T1E1, SiO₂).

### Beispiel 7A

### 3-Amino-2-(2-fluorbenzyl)-pyrazol

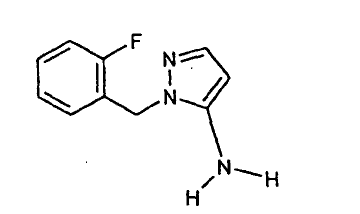

Wurde erhalten analog zum Verfahren beschrieben im Patent Fr. 1403372 (Chem. Abstr. 1965, 63, 14871a).

### Beispiel 8A

### 1-(2-Fluorbenzyl)-pyrazolo[3,4-b]pyrimidin

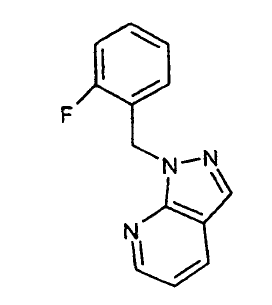

32 g 3-Amino-2-(2-fluorbenryl)-pyrazol werden in 1,5 l Dioxan gelöst und mit 31,45 g Dimethylaminoacrolein versetzt. Man erwärmt auf 50°C und setzt dann 16,65 g Trifluoressigsäure hinzu. Man kocht 60 Stunden, verdampft anschließend das Lösungsmittel im Vakuum, versetzt mit Wasser und extrahiert mit Essigester. Die organische Phase wird mit Na₂SO₄ getrocknet, im Vakuum eingedampft und auf Kieselgel chromatographiert. Man erhält nach Elution mit Toluol →Toluol/Essigester 9:1 17,3 g (46,3 % d. Th.) der Titelverbindung mit einem R_{f} von 0,69 (SiO₂, T₁E₁).

### Beispiel 9A

### 3-Brom-1-(2-fluorbenzyl)-pyrazolo[3,4-b]pyrimidin

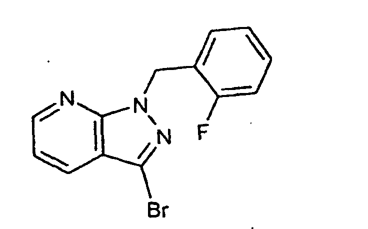

8 g (35,2 mmol) 1-(2-Fluorbenzyl)-pyrazolo[3,4-b]pyrimidin werden in 284 ml Chloroform gelöst und bei Raumtemperatur langsam mit 14 g (87,3 mmol) Brom versetzt. Man rührt über Nacht und tropft dann weitere 1,2 ml Brom hinzu. Nach 2 h wird die Reaktion abgebrochen und im Vakuum eingedampft. Der Rückstand wird mit 20 ml Essigester versetzt und zur Kristallisation gebracht. Nach Waschen der Kristalle mit Ether erhält man 7,5 g (70 % d. Th.) der Titelverbindung mit einem R_{f} von 0,2 (SiO₂, Toluol).

### Beispiel 10A

### 1-(2-Fluorbenzyl)-3-trimethylstannyl-pyrazolo[3,4-b]pyrimidin

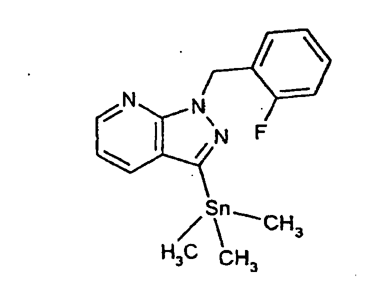

1,22 g (4 mmol) 3-Brom-1-(2-fluorbenzyl)-pyrazolo[3,4-b]pyrimidin werden unter Argon in 200 ml Dioxan gelöst und mit 4,5 g (13,74 mmol) Hexamethyldistannan und 1,2 g Tetrakis(triphenylphosphin)palladium versetzt. Man rührt über Nacht bei 100°C, gibt in Wasser und extrahiert mit Essigester. Die organische Phase wird mit Na₂SO₄ getrocknet, im Vakuum verdampft und auf Kieselgel chromatographiert. Man erhält nach Elution mit Toluol 1,4 g (89,7 % d. Th.) der Titelverbindung mit einem R_{f} von 0,074 (SiO₂, Toluol).

### Beispiel 11A

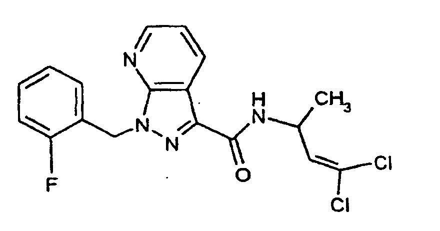

500 mg 1-(2-Fluorbenzyl)-pyrazolo[3,4b]pyridin-3-carbonsäure (1,84 mmol) werden in 10 ml Methylenchlorid angelöst. Man gibt 400 mg N-(3-Dimethylaminopropyl)-N'-ethylcarbodiimid Hydrochlorid (2,3 mmol) hinzu. Man rührt 10 min bei RT (vollständige Lösung) dann tropft man eine Lösung von 260 mg 1,1-Dichlor-3-amino-but-1-en (1,84 mmol) in 5 ml Methylenchlorid hinzu.

Nach etwa 3 h wird die Mischung eingeengt und über Kieselgel (LM:Cyclohexan / EE 1:1) gereinigt. Man erhält 340 mg (47 %) 1-(2-Fluorbenzyl)-3-(1,1-dichlorbut-1-en-3-yl-amido)-pyrazolo[3,4]pyridin, Rf 0,35 (Cyclohexan:EE 2:1). MS (ESI-POSITIV): 417 (27, [M+Na]⁺); 415 (42, [M+Na]⁺); 395 (60, [M+H]⁺); 393 (100, [M+H]⁺).

### Herstellungsbeispiele

### Beispiel 1 (Referenzbeispiel)

### 1-(2-Fluorbenzyl)-3-(2-hydroxymethyl-2-furyl)-pyrazolo-[3,4-b]-pyrazin

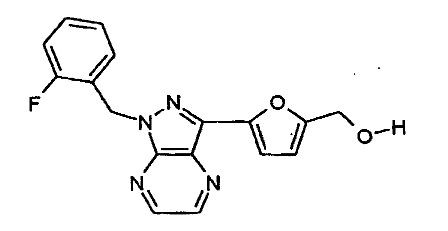

Der Rohansatz (8 g in 200 ml Ethanol) aus Beispiel 6A wird mit 4,61 g einer 40%igen wäßrigen Glyoxallösung versetzt und 10 h bei Raumtemperatur gerührt Die Mischung wird im Vakuum eingedampft und über SiO₂ mit Toluol/Essigestergemischen chromatographiert. Nach Kristallisation mit Ether erhält man 0,57 g (7,6 % d.Th.) der Titelverbindung mit einem Smp. von 194°C.

### Beispiel 2 (Referenzbeispiel)

### 1-(2-Fluorbenzyl)-3-(2-furyl)-6-hydroxy-pyrazolo[3,4-b]pyridin

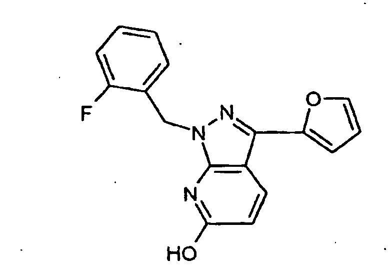

7 g (27,2 mmol) 5-Amino-1-(2-fluorbenzyl)-3-(2-furyl)-pyrazol, 3,94 g (27,3 mmol) 3-Ethoxyacrylsäureethylester und 1,96 ml (27,3 mmol) Trifluoressigsäure werden innig vermischt und in einem Mikrowellenofen 2 Minuten lang zur Reaktion gebracht. Die Mischung wird in eine Lösung von 10 g K₂HPO₄ in 500 ml Wasser gegeben und mit 500 ml Essigester extrahiert. Nach Trocknung der organischen Phase mit MgSO₄ gibt man 30 g Kieselgel hinzu und verdampft im Vakuum. Der Rückstand wird über eine Kieselgelsäule mit einem Toluol-Essigester-Gradienten chromatographiert. Die erste Fraktion wird mit Ether kristallisiert und ergibt 1,8 g (21,4 % d.Th.) der Titelverbindung mit einem Smp. von 250°C.

In Analogie zur Vorschrift des Beispiels 2 wurden die in der Tabelle 1 aufgeführten Verbindungen hergestellt:

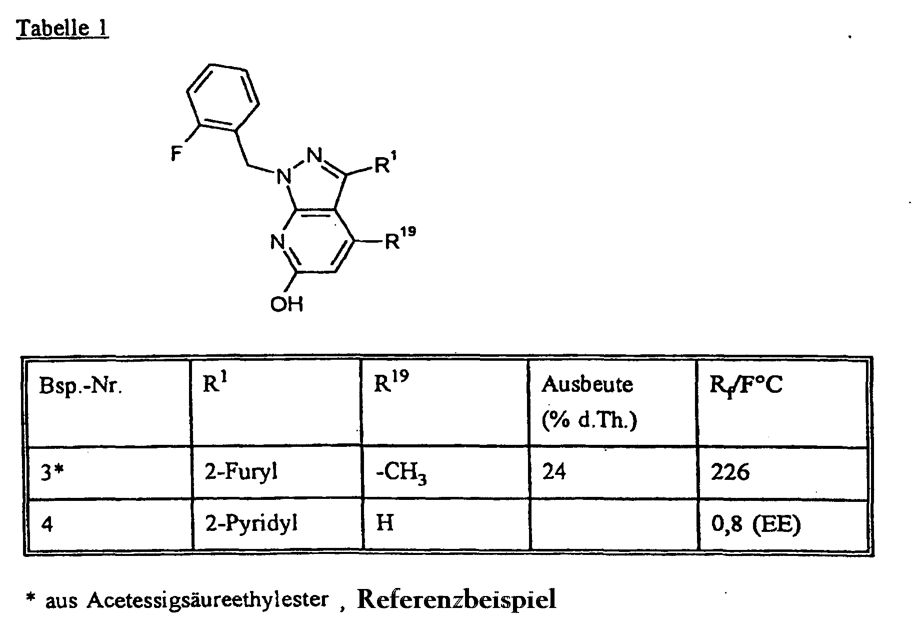

### Beispiel 5 (Referenzbeispiel)

### 4-Amino-1-(2-fluorbenzyl)-3-(2-furyl)-pyrazolo[3,4-d]pyrimidin

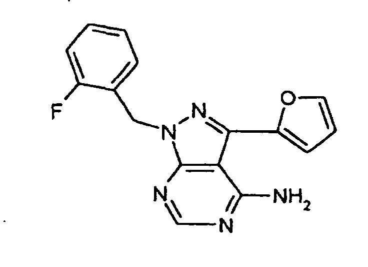

2 g (7,1 mmol) 5-Amino-4-cyano-1-(2-fluorbenryl)-3-(2-furyl)-pyrazol und 30 ml Formamid werden erst bei 100°C und dann 3 h bei 195°C gerührt. Die beim Abkühlen kristallisierende Masse wird abgesaugt, mit kaltem Formamid gewaschen, der Rückstand in Essigester aufgenommen und mit Wasser gewaschen. Nach Trocknen und Verdampfen der organischen Phase erhält man 2,13 g (97 % d.Th.) der Titelverbindung mit einem Smp. von 190°C. R_{f} = 0,07 (TIE1).

In analoger Weise erhält man z.B. aus 2-Propyloxyphenylnitril, Benzonitril bzw. Guanidiniumhydrogencarbonat die in der Tabelle 2 aufgeführten Verbindungen:

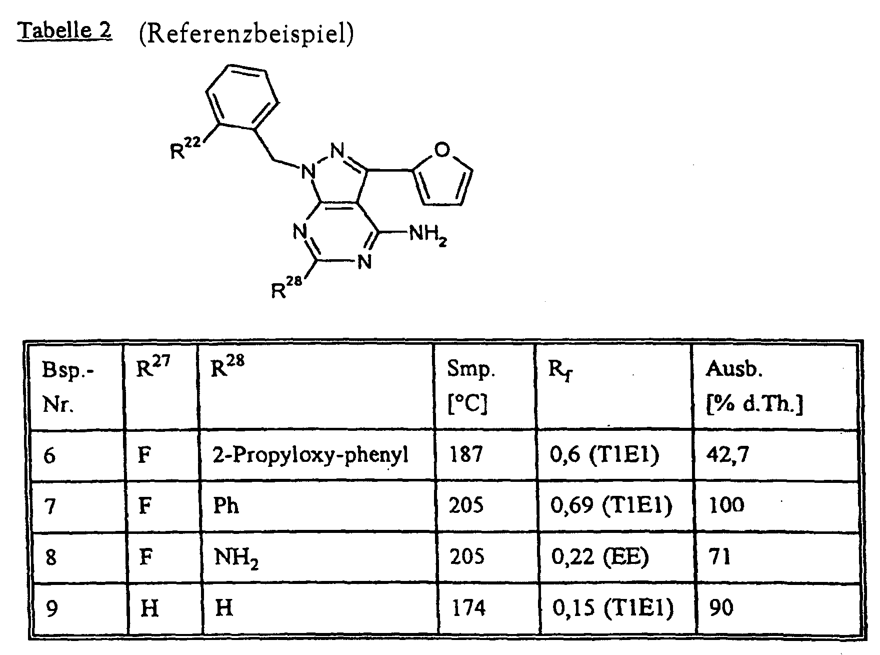

### Beispiel 10 (Referenzbeispiel)

### 1-Benzyl-3-(2-furyl)-4-hydroxypyrazolo[3,4-d]pyrimidin

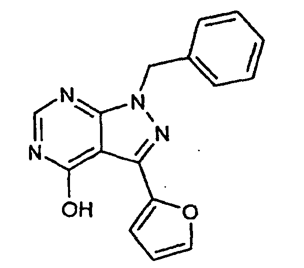

6.14 g (23.2 mmol) 5-Amino-1-benzyl-4-cyano-3-(2-furyl)-pyrazol (R_{f} = 0.6) werden in 100 ml Ameisensäure 3.75 h gekocht. Anschließend wird der Ansatz im Vakuum eingedampft. Man versetzt mit Wasser und schüttelt mit Essigester aus. Der unlösliche Teil wird abgesaugt und ergibt 5.1 g der Zielverbindung (Smp. = 242°C, R_{f} = 0.3, SiO₂, Toluol-Essigester = 1:1). Durch Eindampfen der organischen Phase können weitere Mengen isoliert werden.

### Beispiel 11 (Referenzbeispiel)

### 1-Benzyl-4-chlor-3-(2-furyl)-pyrazolo[3,4-d]pyrimidin

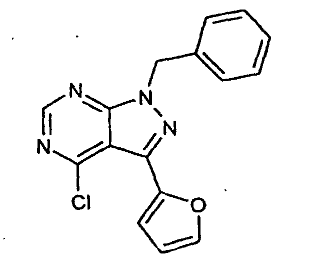

6.6 g 1-Benzyl-3-(2-furyl)-4-hydroxypyrazolo[3,4-d]pyrimidin werden 12 h lang in 100 ml POCl₃ gekocht. Man dampft im Vakuum ein, verrührt mit wässriger K₂HPO₄-Lösung und extrahiert mit Essigester. Nach Trocknen mit Na₂SO₄ und Einrotieren erhält man 7.47 g eines Feststoffs, der für die nächste Stufe direkt umgesetzt werden kann (R_{f} = 0.8, SiO₂, Toluol-Essigester = 1:1).

### Beispiel 12 (Referenzbeispiel)

### 1-Benzyl-3-(2-furyl)-pyrazolo[3,4-d]pyrimidin

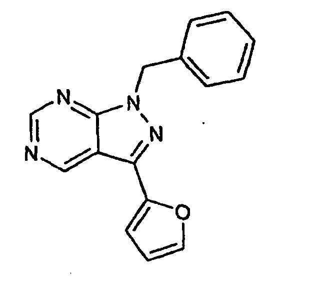

5.81 g 1-Benzyl-4-chlor-3-(2-furyl)-pyrazolo[3,4-d]pyrimidin werden in 450 ml Dioxan gelöst und mit 4 g 20-proz. Pd(OH)₂ auf Kohle nach Zugabe von 2.61 ml Triethylamin 5h bei 3 bar Wasserstoffdruck in der Parr-Apparatur hydriert. Nach Filtration durch Kieselgur, Eindampfen und Chromatographieren erhält man 2.26 g gelbliche Kristalle (Smp. = 106°C, R_{f}= 0.2 Toluol-Essigester = 4:1).

### Beispiel 13 (Referenzbeispiel)

### 1-Benzyl-3-(5-hydroxymethyl-2-furyl)-1-H-pyrazolo[3,4-b]pyridin

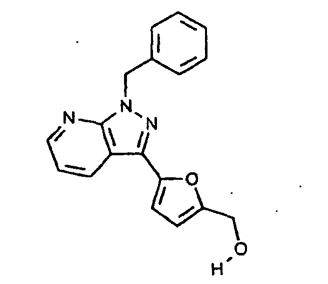

2.69 g (10 mmol) 5-Amino-1-benzyl-3-(5-hydroxymethyl-2-furyl)-pyrazol und 1.4 g Malondialdehydhydratnatriumsalz werden in 100 ml Dioxan bei 100°C 30 min gerührt und während 5.5 Std. langsam mit 1.9 ml Trifluoressigsäure versetzt. Man verdampft im Vakuum, nimmt in Essigester auf, schüttelt mit K₂HPO₄-Lösung aus, trocknet die organische Phase mit Na₂SO₄ und rotiert im Vakuum ein. Der Rückstand wird auf Kieselgel chromatographiert. Man erhält 200 mg (6.6 % d. Th.) Kristalle mit einem Schmelzpunkt von 104°C.

In Analogie zu den oben aufgeführten Vorschriften werden die in der Tabelle 3 genannten Verbindungen hergestellt:

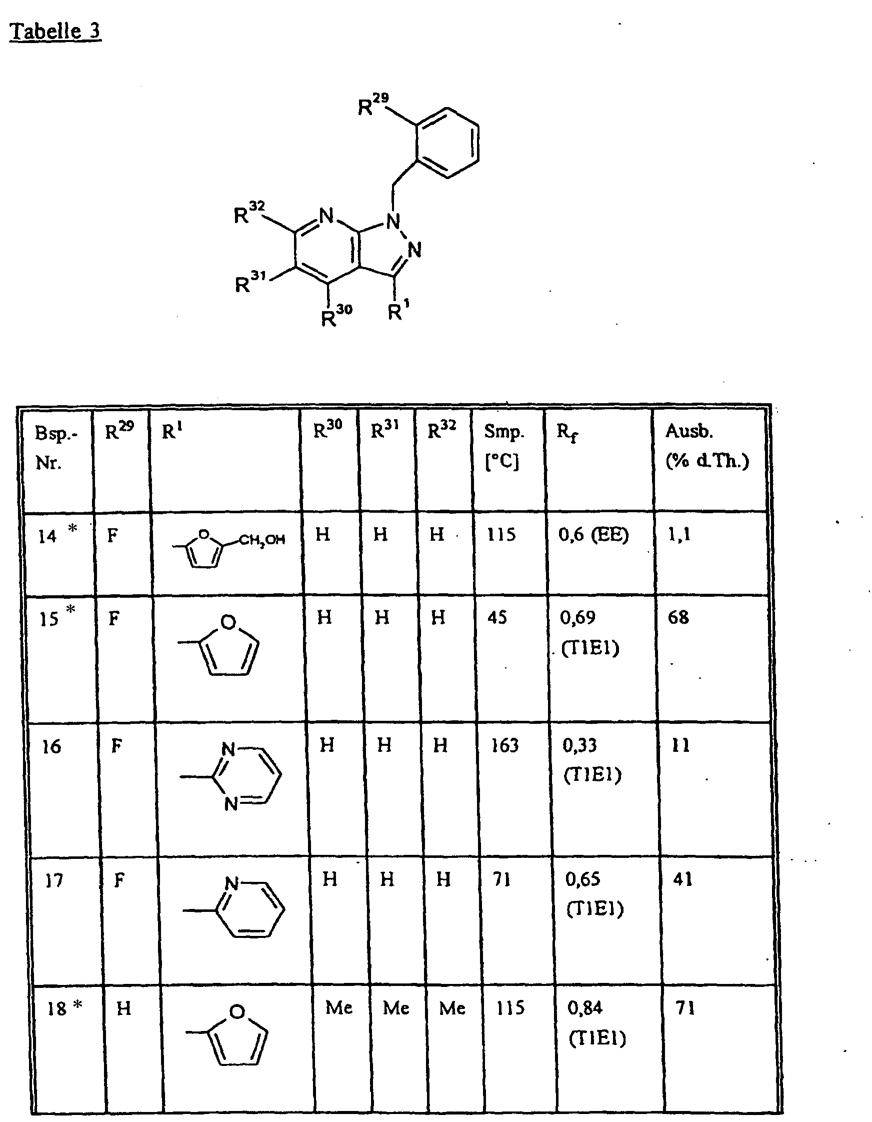

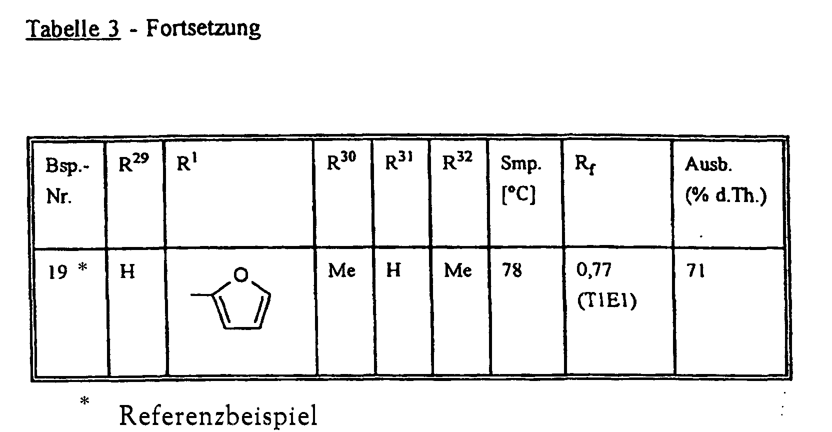

### Beispiel 20

### 3-(4,5-Dimethylpyrimidin-2-yl)-1-(2-fluorbenzyl)-pyrazolo[3,4-b]pyrimidin

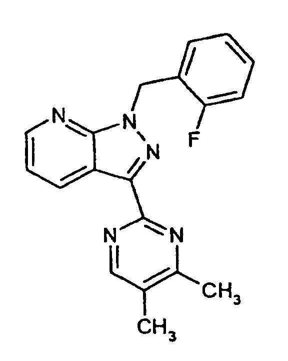

1,4 g (3,59 mmol) 1-(2-Fluorbenryl)-3-trimethylstannyl-pyrazolo[3,4-b]pyrimidin werden in Toluol unter Argon mit 0,51 g (3,58 mmol) 2-Chlor-4,5-dimethylpyrimidin und 0,2 g (0,28 mmol) Bis(triphenylphosphin)dichlorpalladium über Nacht gekocht. Man gibt 3 g Kieselgel hinzu und verdampft das Lösungsmittel im Vakuum. Anschließend chromatographiert man über Kieselgel und eluiert mit einem Toluol/Essigester-Gemisch. Man erhält 0,34 g (28,4 % d.Th.) der Titelverbindung mit einem Smp. von 167°C und einem R_{f} von 0,08 (SiO₂, T4E).

Auf analoge Weise wurde das in der Tabelle 4 aufgeführte Beispiel hergestellt:

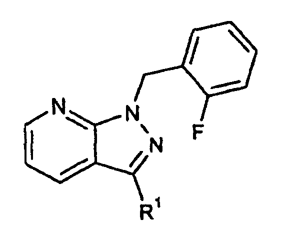

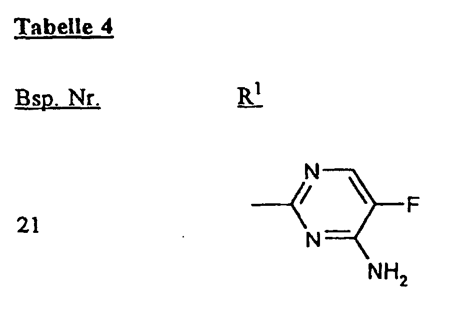

### Beispiel 22 (Referenzbeispiel)

### 1-Benzyl-3-(5-formyl-2-furyl)-pyrazolo[3,4-d]pyrimidin

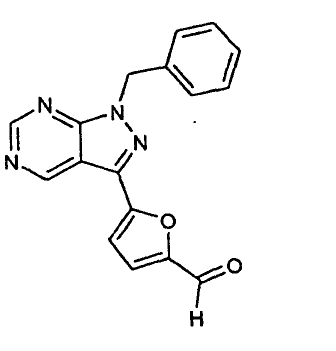

Man gibt bei 0°C 0.74 ml POCl₃ zu 0.64 ml DMF. Die erstarrte Mischung läßt man auf Raumtemperatur kommen und gibt 14 ml 1,2-Dichlorethan hinzu. In diese Lösung tropft man bei 15°C eine Lösung von 2 g 1-Benryl-3-(2-furyl)-pyrazolo[3,4-d]pyrimidin (R_{f} = 0.45, SiO₂, Toluol-Essigester = 1:1) in 14 ml 1,2-Dichlorethan und erwärmt dann auf 80°C. Nach 4 h wird der gesamte Ansatz zu weiterem Vilsmeier-Reagens, das aus 1.5 ml POCl₃ und 1.3 ml DMF hergestellt wurde, getropft und 24 h bei 80°C gerührt. Anschließend gibt man die Mischung in eine 50-proz. wässrige Lösung von K₂HPO₄ und erhitzt kurz unter Rühren auf 75°C. Nach Extraktion mit Essigester, Trocknen der organischen Phase, Einrotieren und Chromatographie auf SiO₂ erhält man 0.6 g (27 % d. Th.) eines Öls (R_{f} = 0.3, SiO₂, Toluol-Essigester = 1:1).

In Analogie zu den oben aufgeführten Vorschriften werden die Beispiele aus der Tabelle 5 hergestellt.

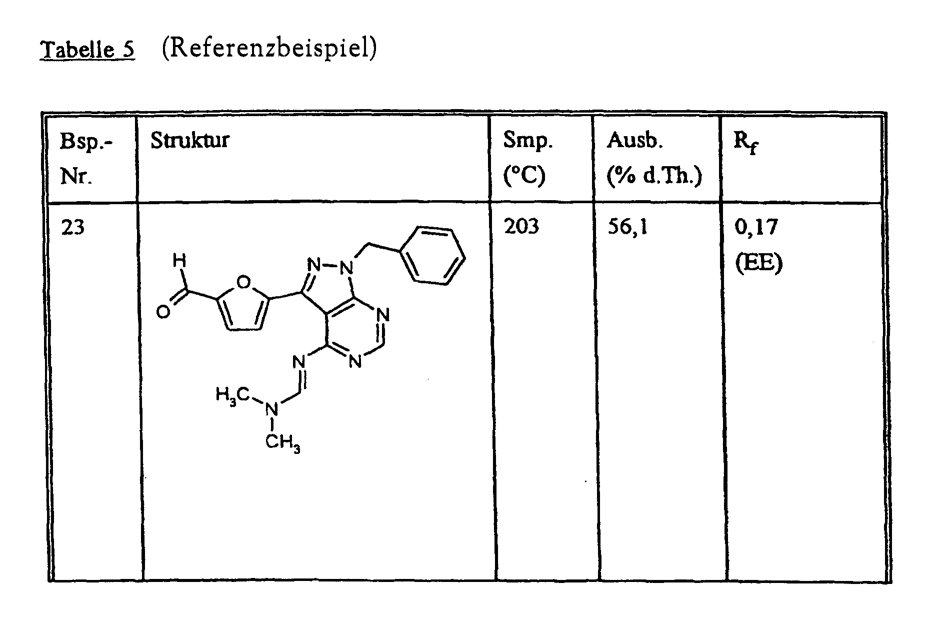

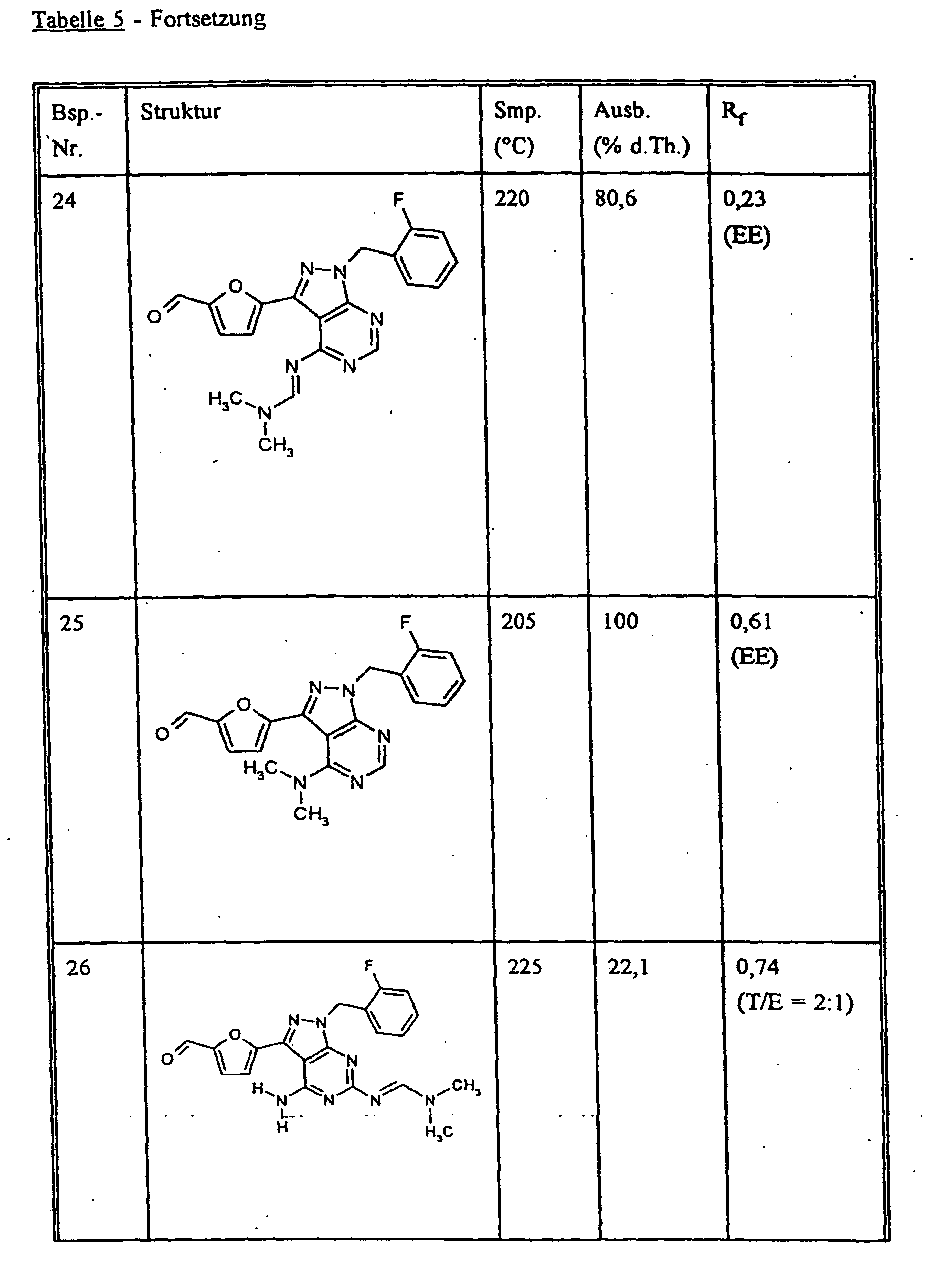

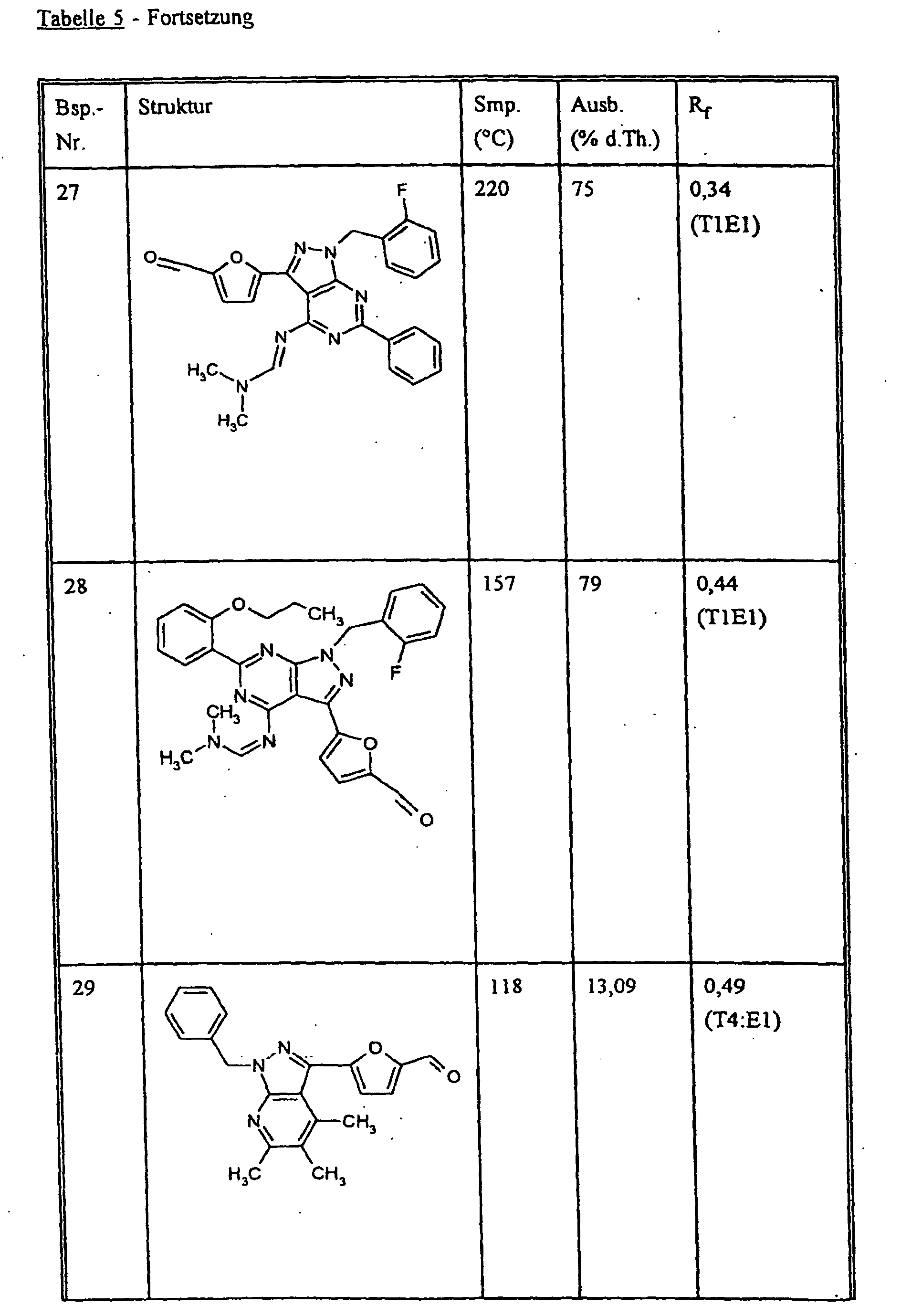

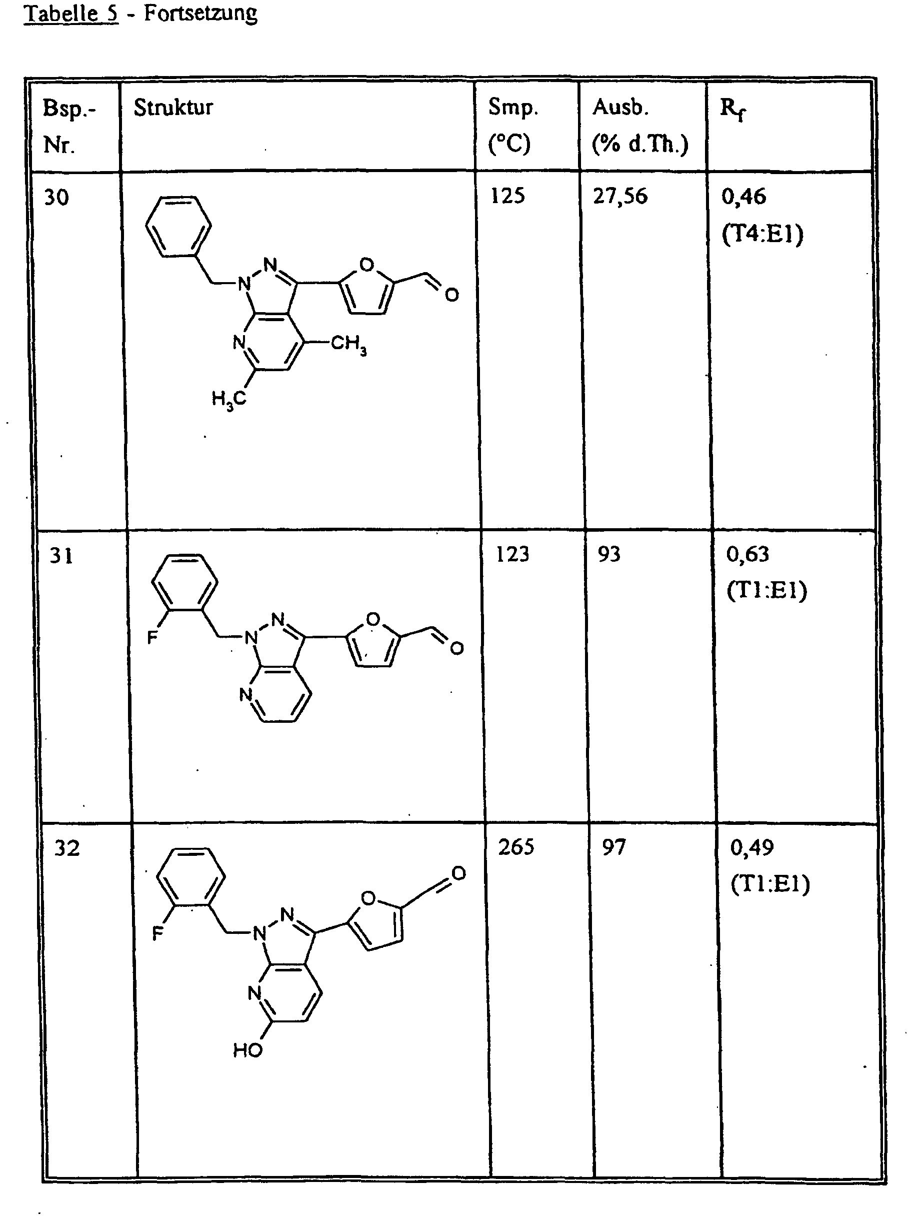

### Beispiel 33 (Referenzbeispiel)

### 1-Benzyl-3-(5-hydroxymethyl-2-furyl)-pyrazolo[3,4-d]pyrimidin

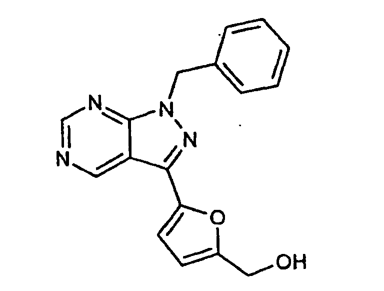

0.6 g (1.97 mmol) 1-Benryl-3-(5-formyl-2-furyl)-pyrazolo[3,4-d]pyrimidin (R_{f} = 0.65, SiO₂, Essigester) werden in 20 ml 1-Propanol bei Raumtemperatur unter gutem Rühren mit 60 mg NaBH₄ versetzt. Nach 15 min gibt man 50 ml Wasser und 2.5 ml Eisessig hinzu. Nach teilweisem Einrotieren wird mit Essigester extrahiert, getrocknet und nach Toluol-Zusatz einrotiert. Nach Chromatographie auf SiO₂ erhält man 74.8 mg (12.4 % d. Th.) Produkt (Smp. 165°C, R_{f} = 0.43, SiO₂, Essigester).

In Analogie zur Vorschrift des Beispiels 33 werden die in der Tabelle 6 aufgeführten Verbindungen hergestellt:

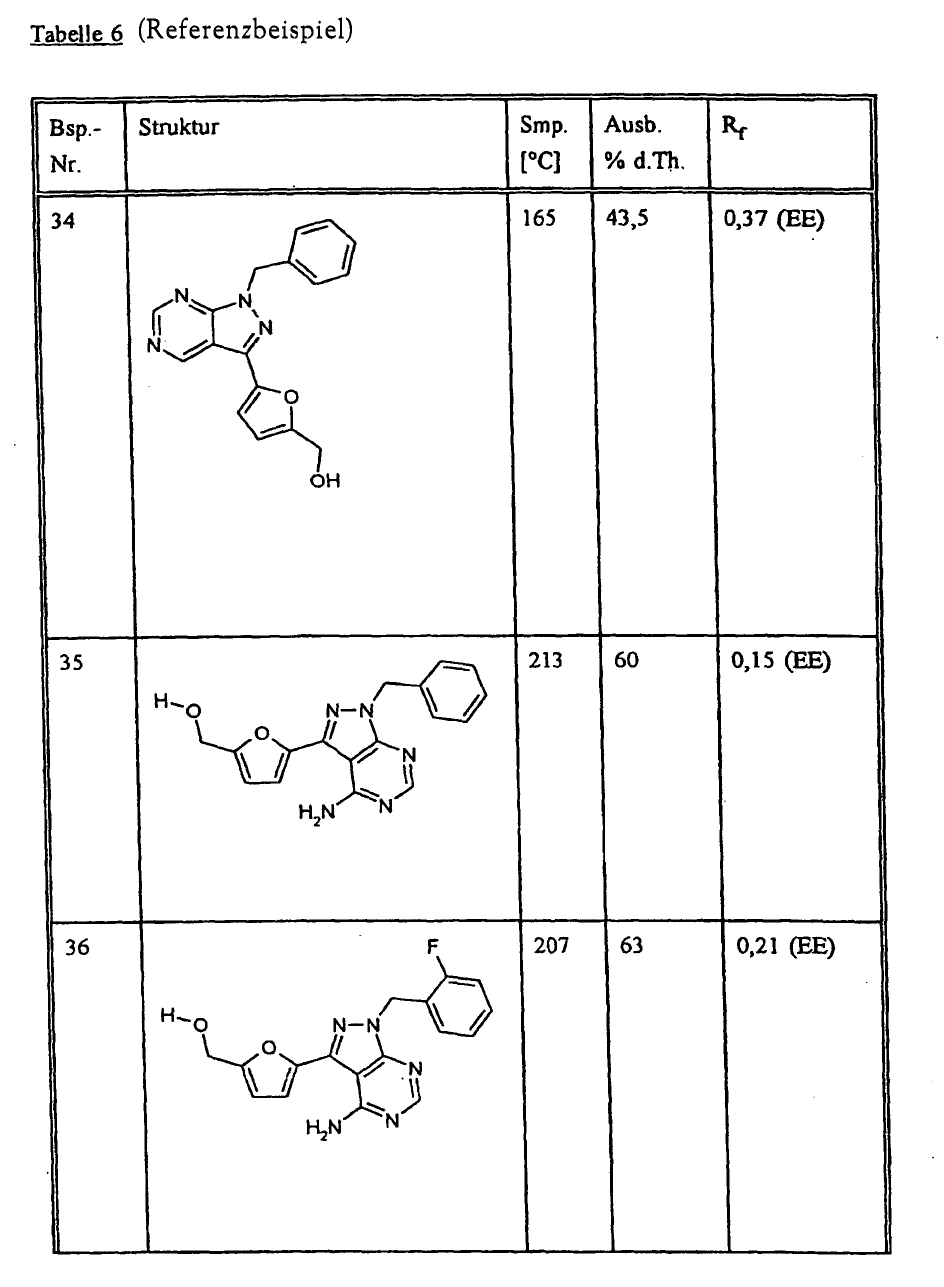

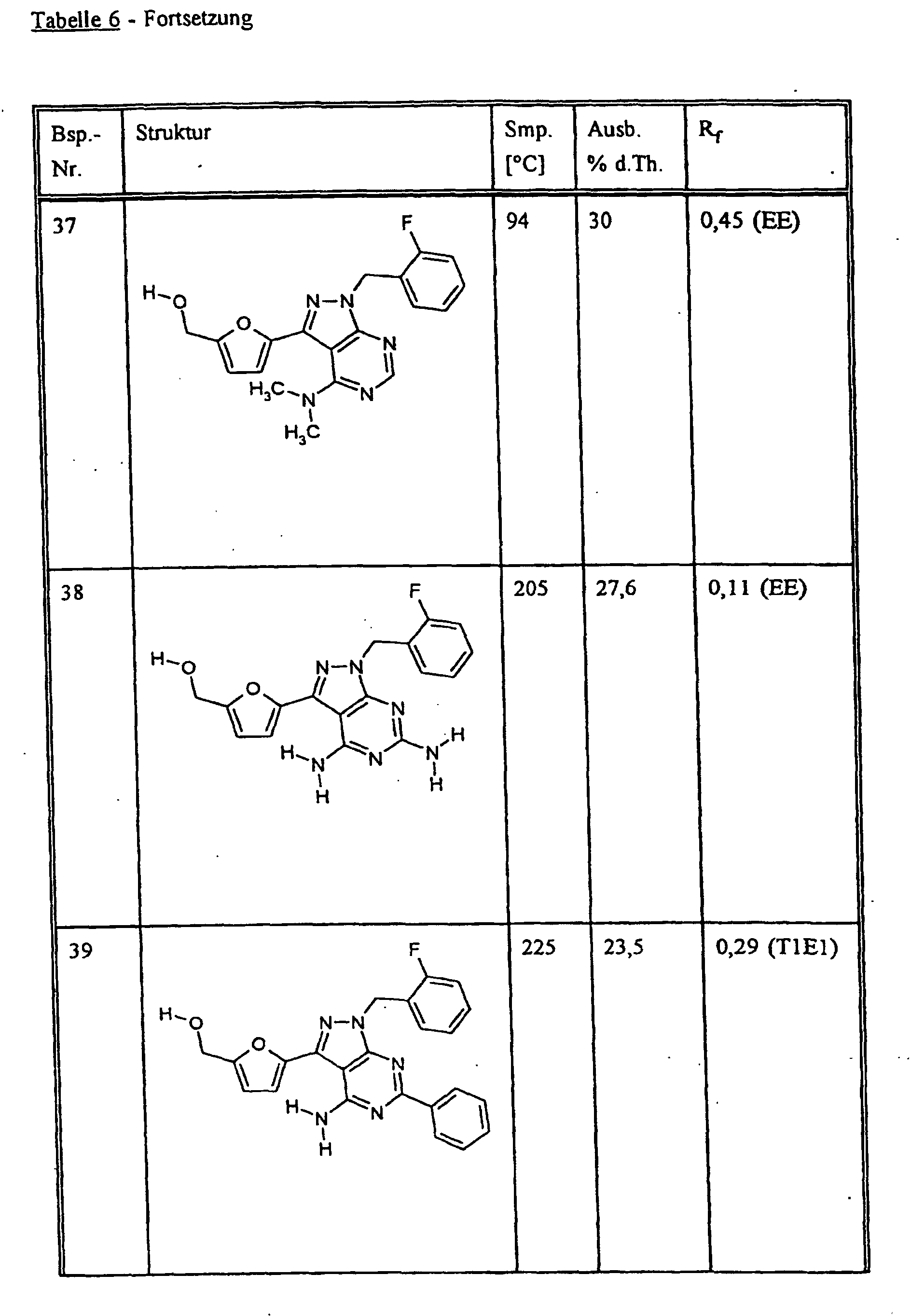

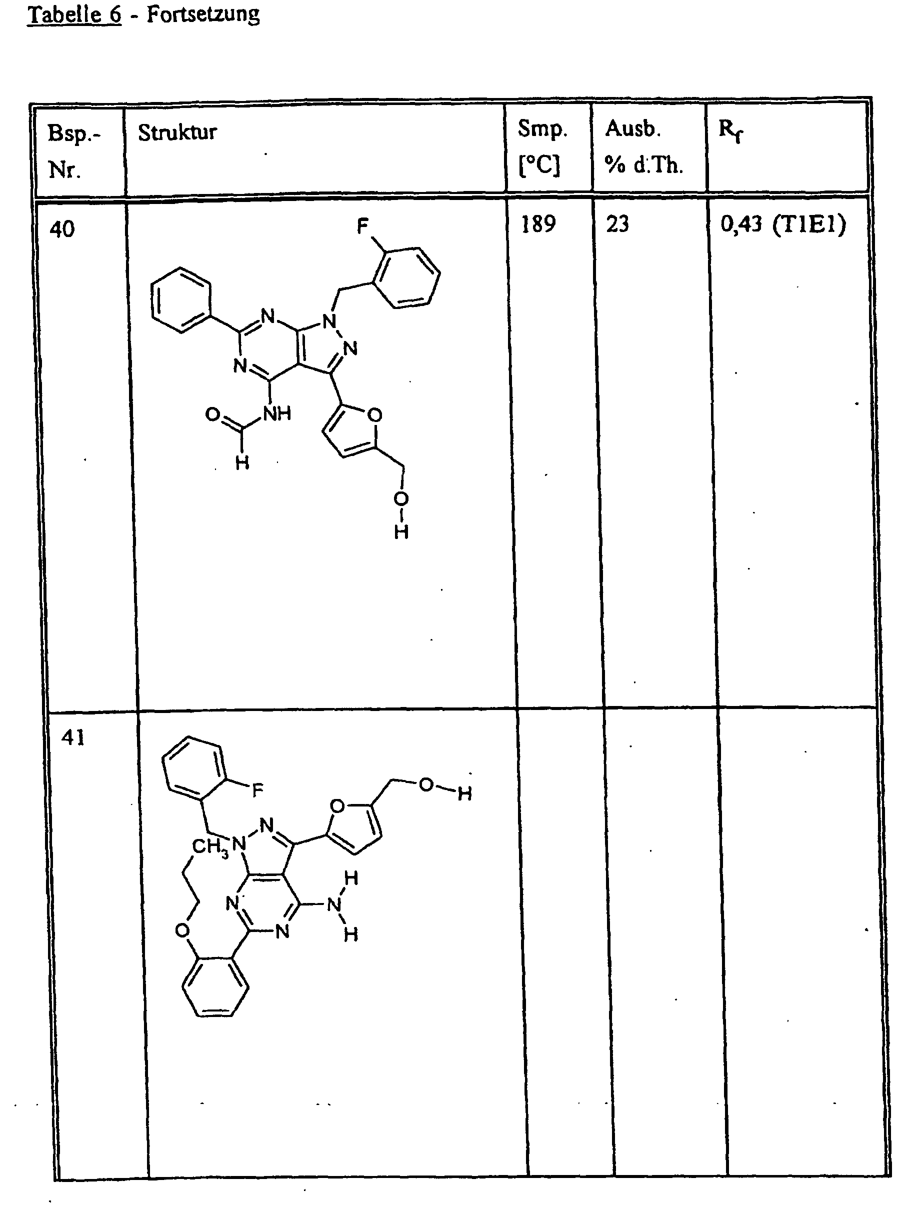

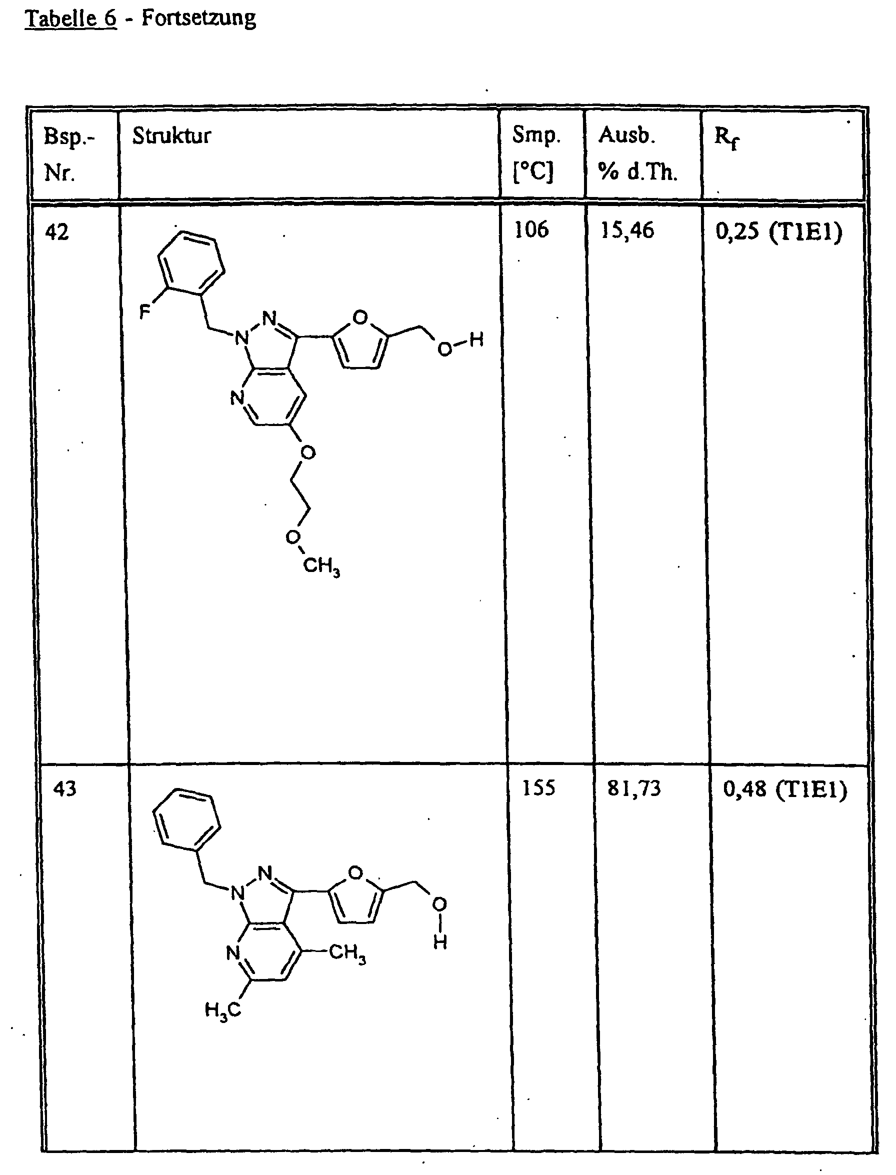

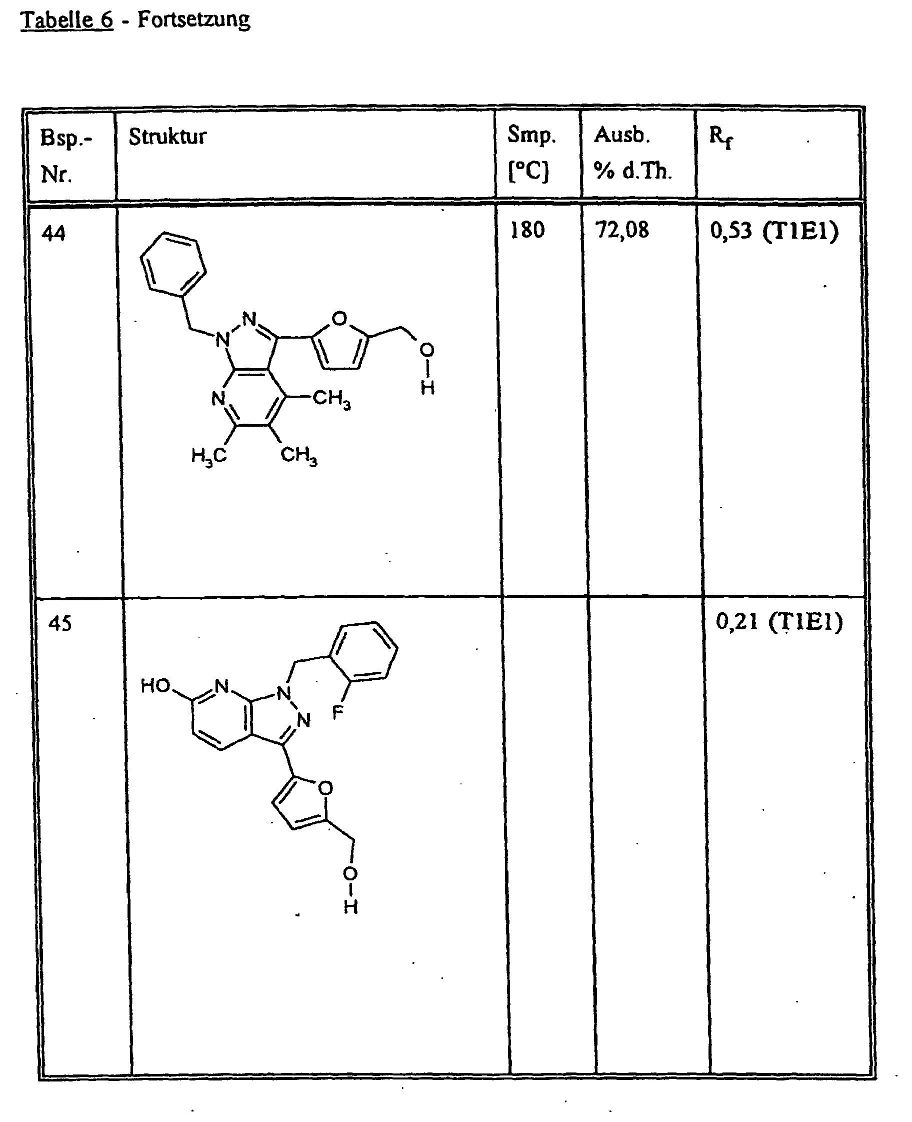

### Beispiel 46 (Referenzbeispiel)

Darstellung von 1-(2-Fluorbenryl)-3-[5-(piperidin-1-sulfinyl)-furan-2-yl]-1Hpyrazolo[3,4-b]pyridin 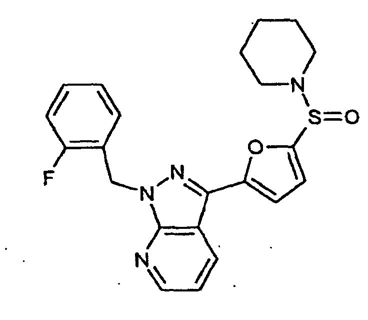
a) Darstellung von 1-(2-Fluorbenryl)-3-[5-chlorsulfinyl-furan-2-yl]pyrazolo-[3,4-b]pyridin 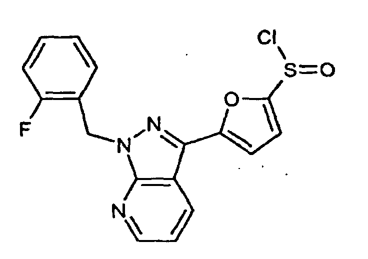 0,85 g (2,86 mmol) 1-(2-Fluorbenzyl)-3-(2-furyl)pyrazolo[3,4-b]pyridin werden 25 min bei 70°C mit 20 ml Thionylchlorid gerührt. Anschließend wird der Ansatz im Vakuum eingedampft und roh weiter umgesetzt.
b) Obiger Ansatz wird in 30 ml Dioxan aufgenommen, mit 0,6 ml (ca. 6 mmol) Piperidin versetzt, kräftig geschüttelt und über Nacht stehen gelassen. Man gibt in Wasser und schüttelt mit Essigester aus. Nach Trocknen der organischen Phase mit Na₂SO₄ wird eingedampft und der Rückstand über Kieselgel mit einem Toluol/Essigester-Gradienten chromatographiert. Man erhält 0,49 g (40 % d.Th.) eines braunen, klaren zähen Sirups (R_{f} (SiO₂; T1E1) = 0,36).

In Analogie zu den oben aufgeführten Vorschriften und der des Beispiels 46 werden die in der Tabelle 7 aufgeführten Verbindungen hergestellt.

Das Beispiel 67 wird in Analogie zur Vorschrift des Beispiels 20 hergestellt.

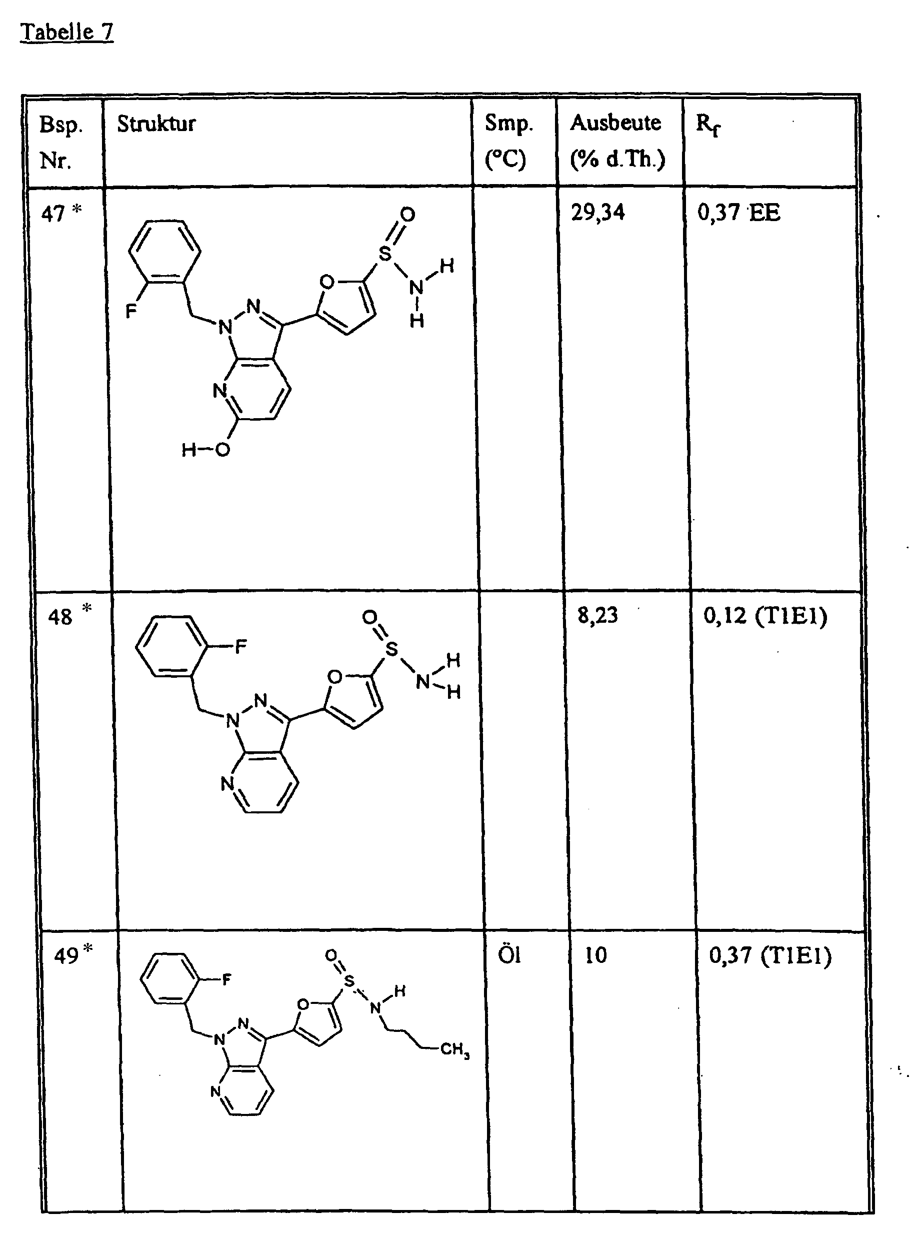

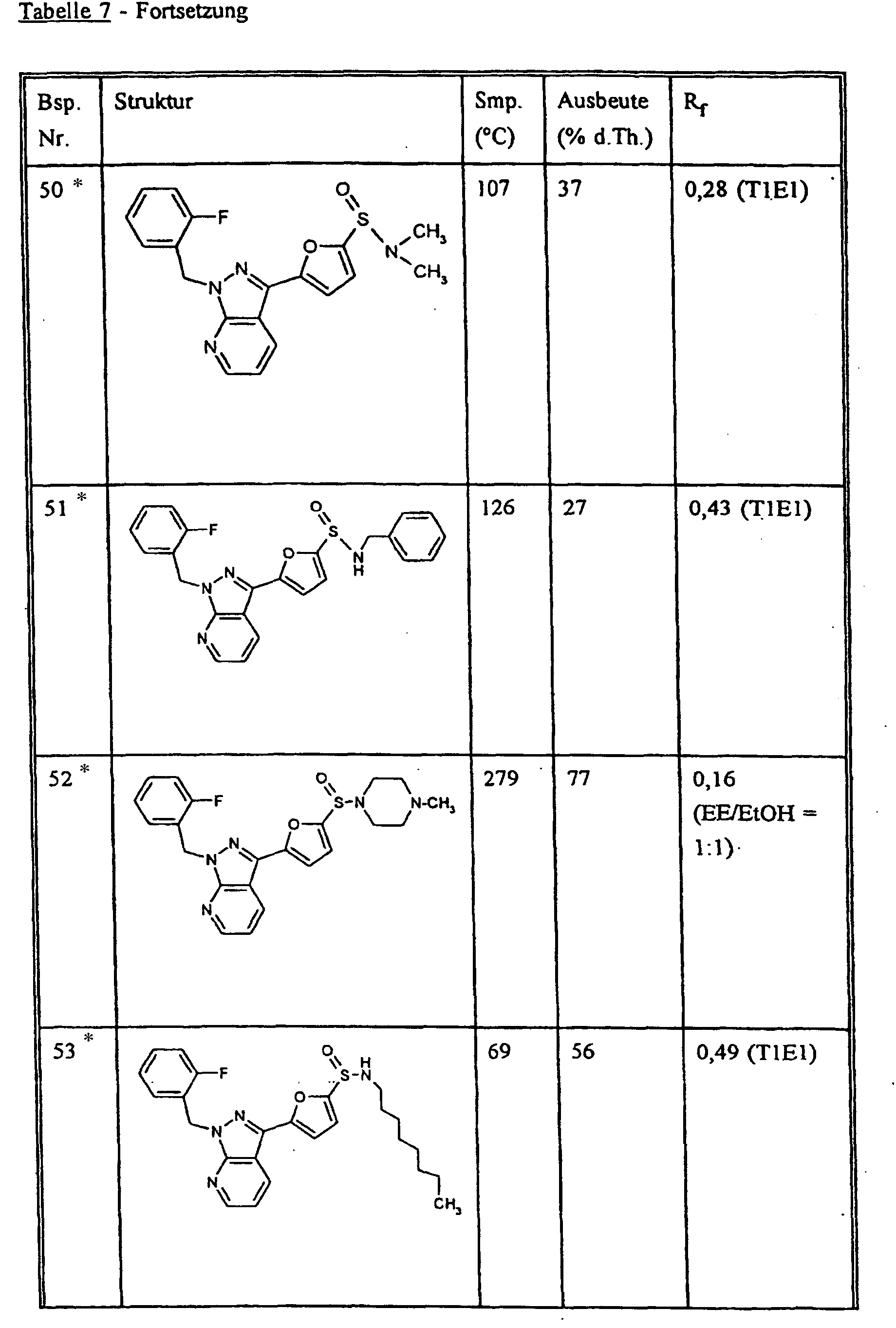

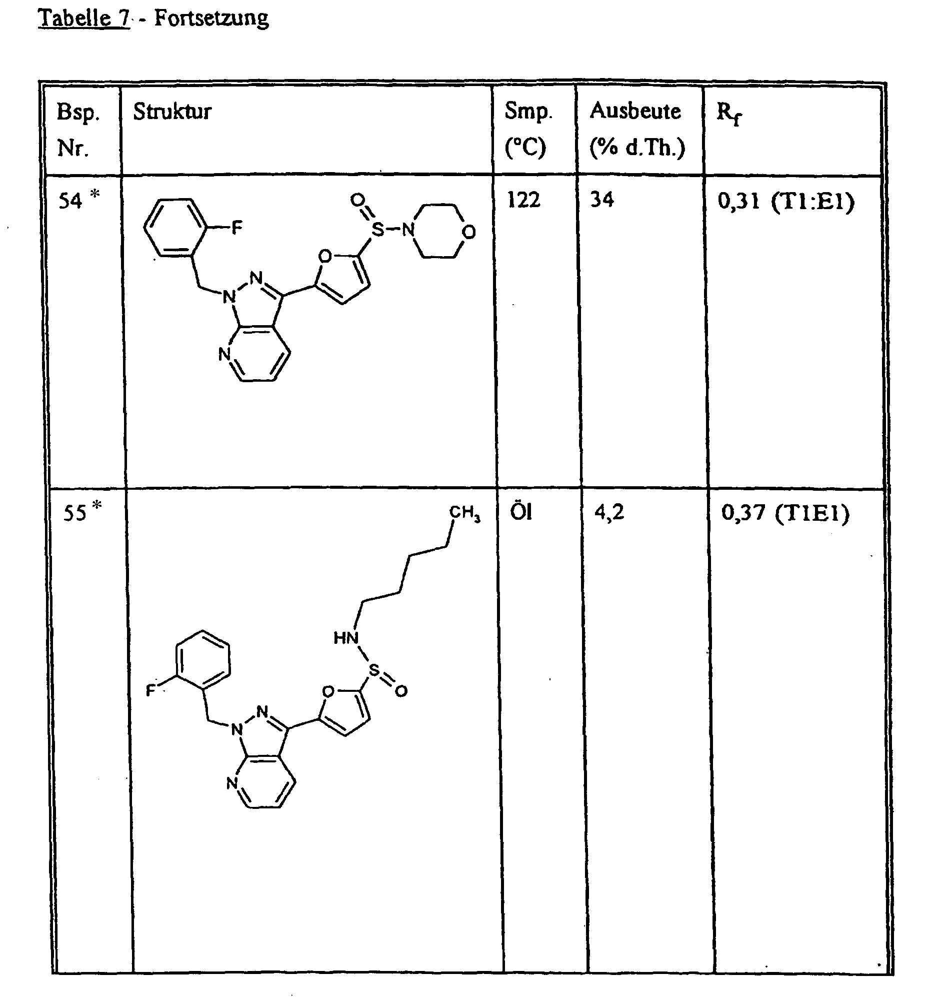

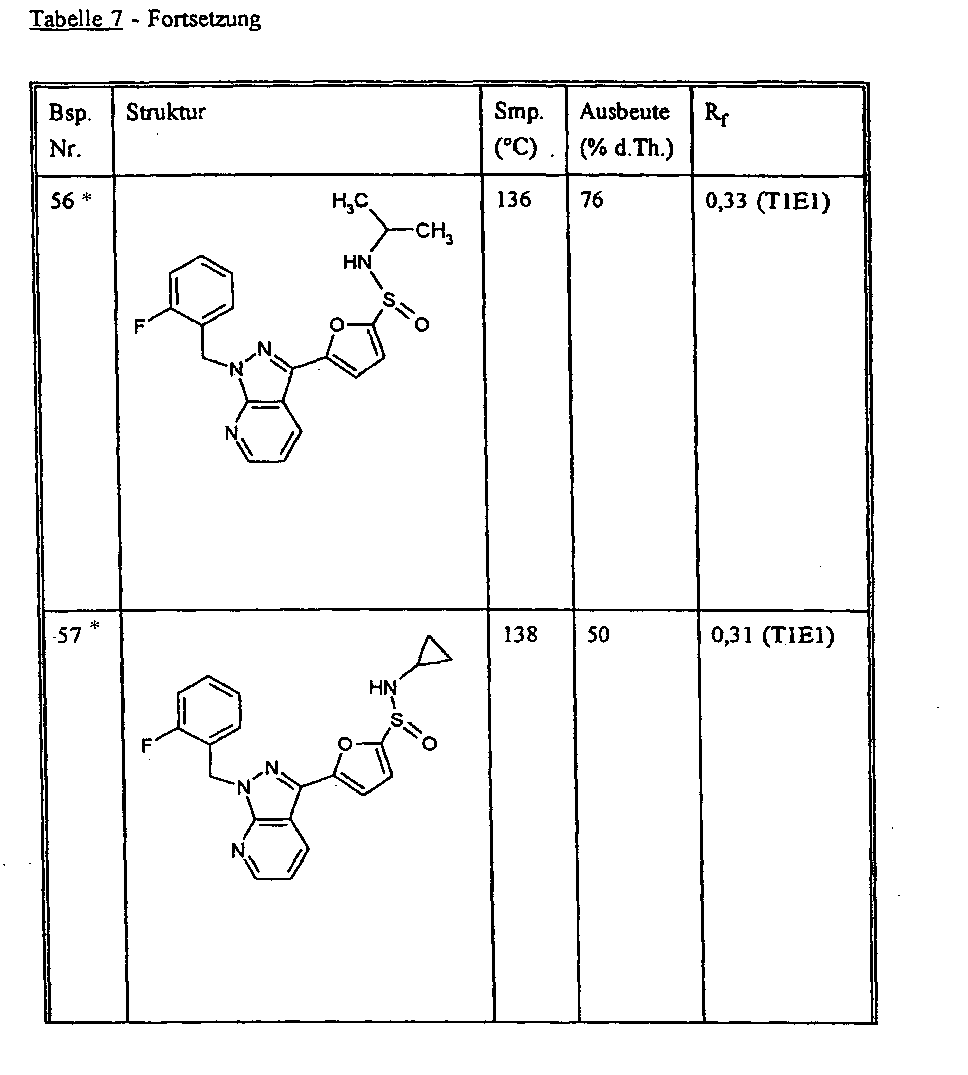

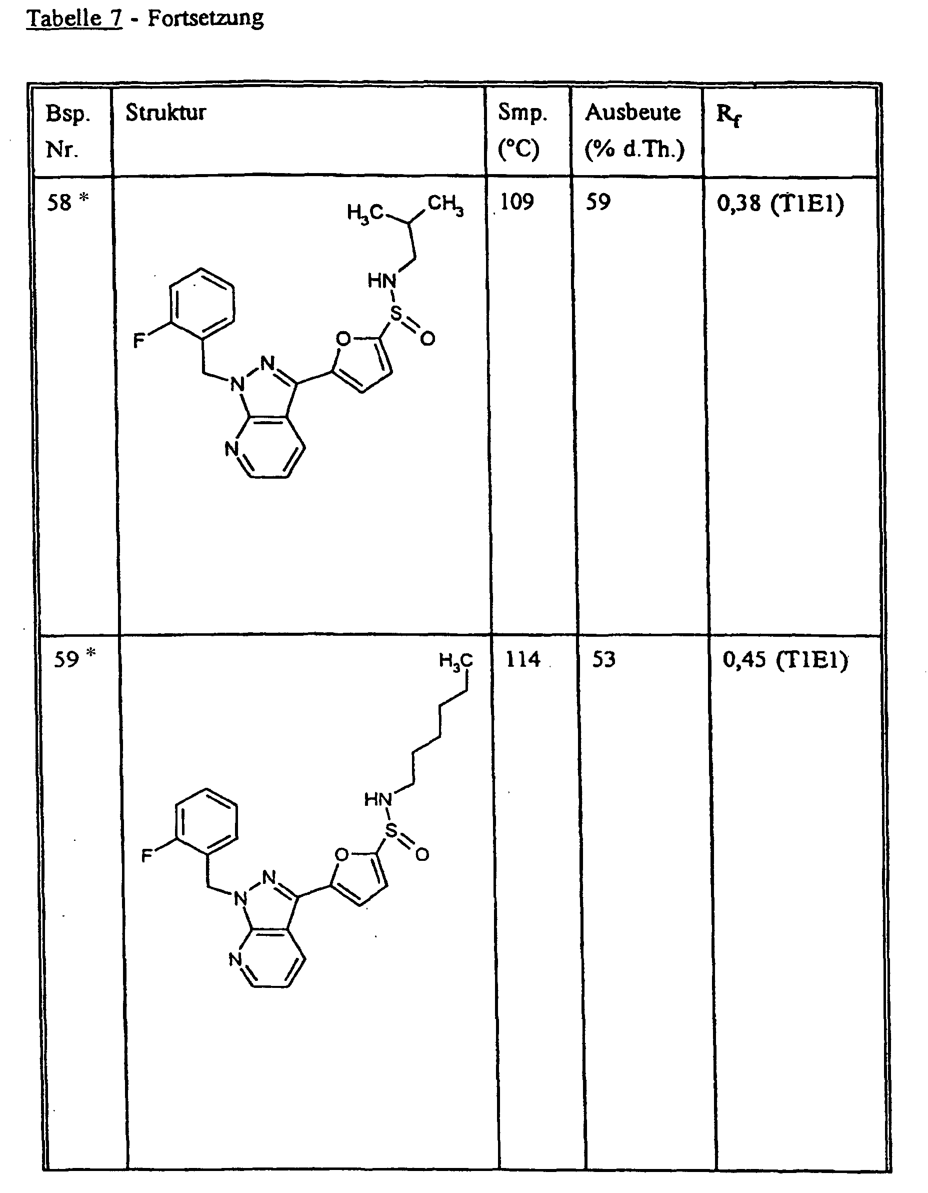

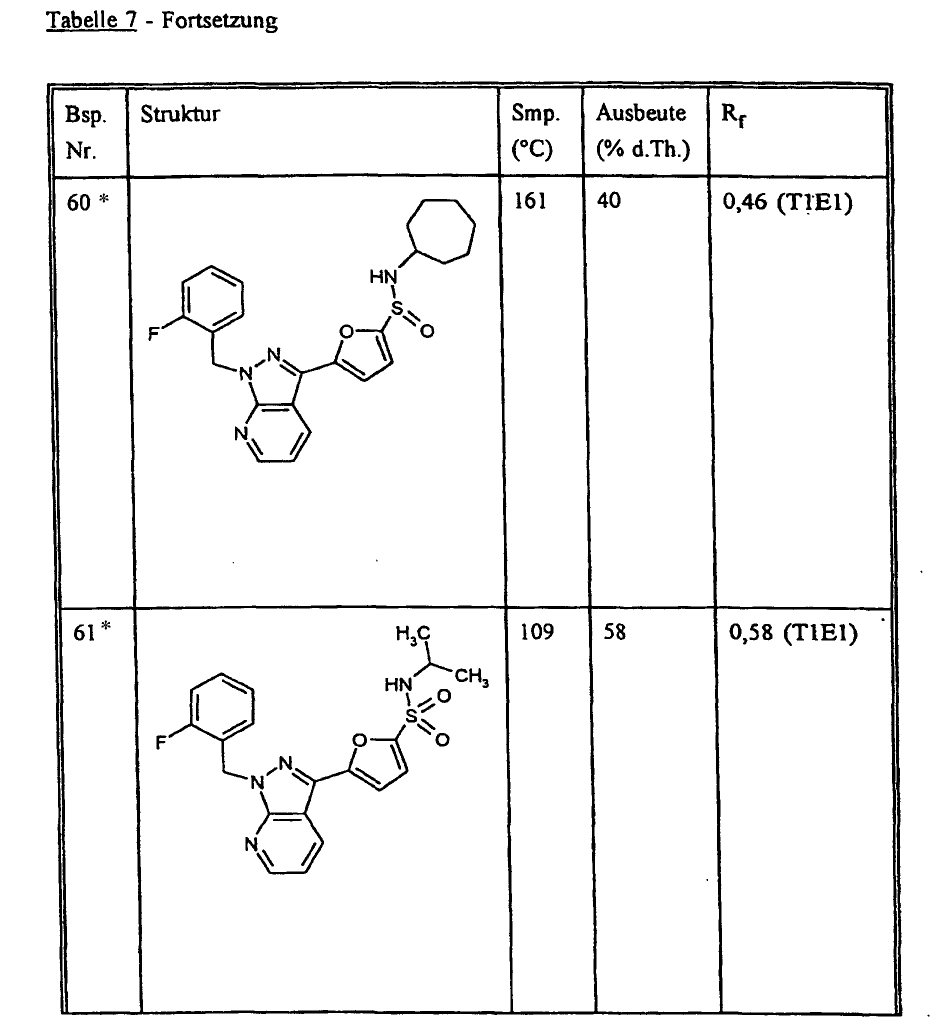

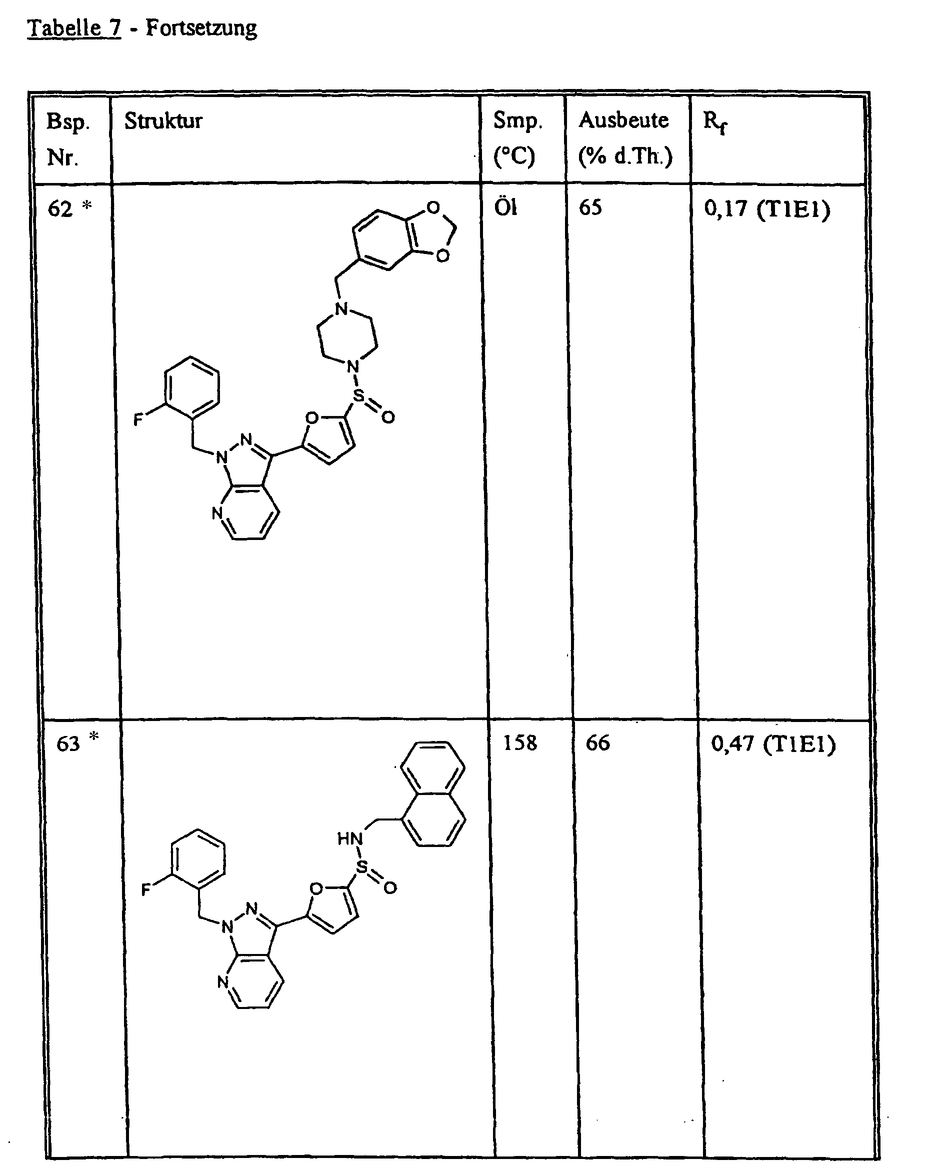

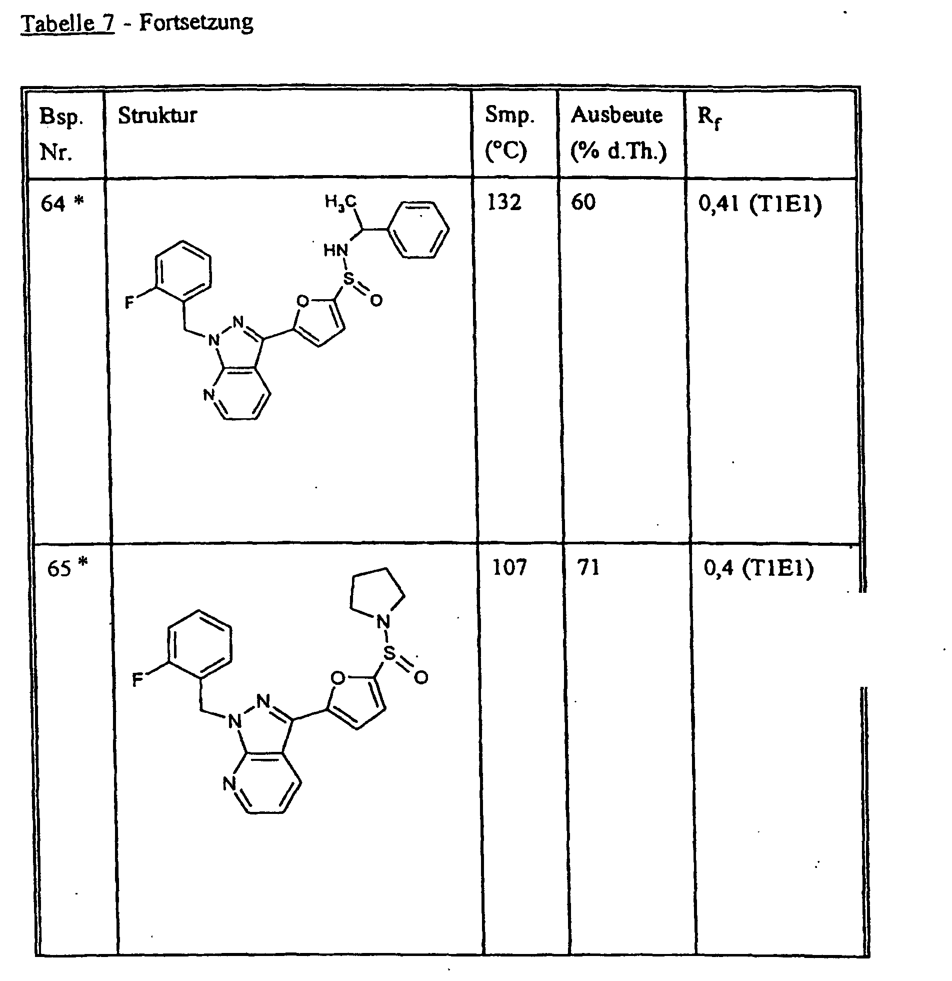

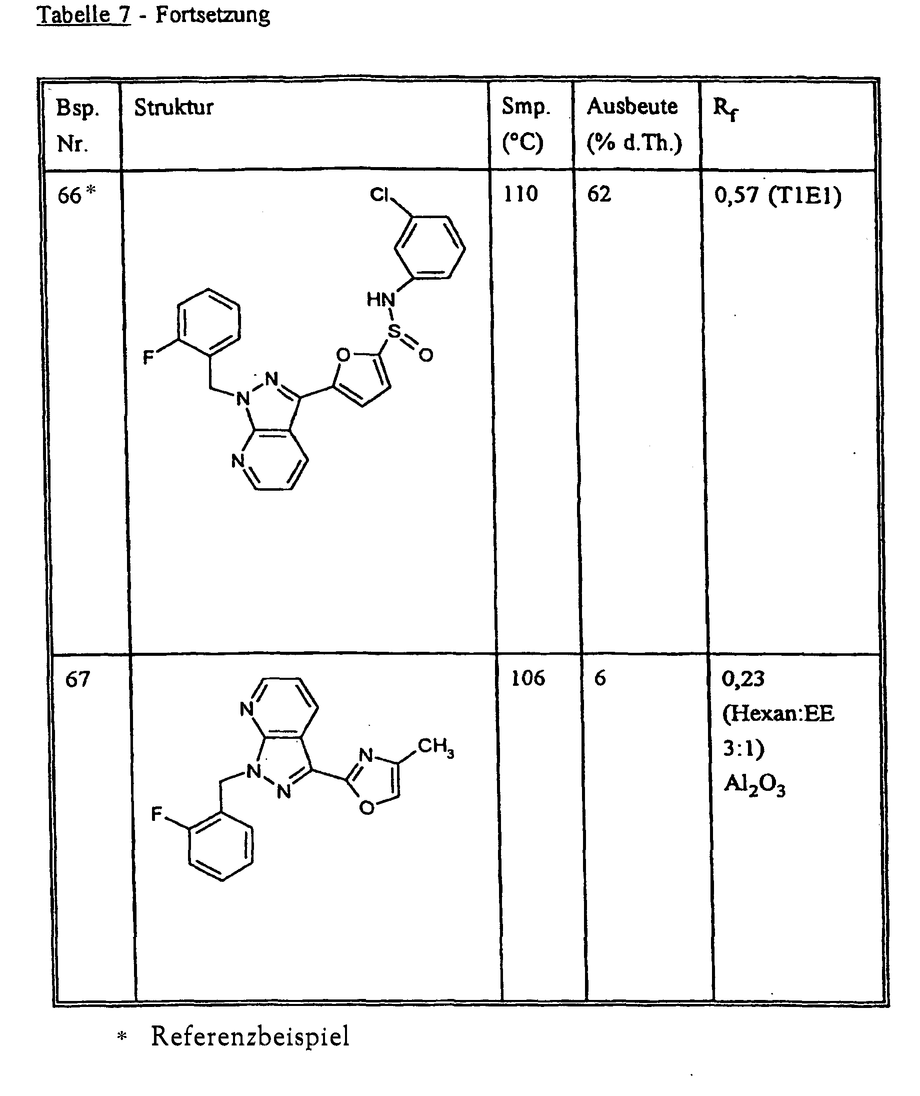

### Beispiel 68

### 1-(2-Fluorbenzyl)-3-(4-methyl-3-hydroxymethyl-oxazol-2-yl)-pyrazolo[3,4b]pyridin

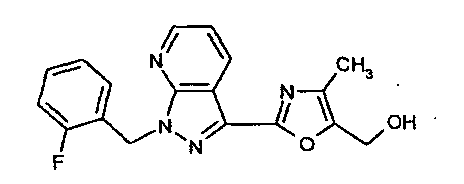

330 mg 1-(2-Fluorbenryl)-3-(1,1-dichlorbut-1-en-3-yl-amido)-pyrazolo[3,4b]pyridin (0,84 mmol), 1,7 ml NaOH 1N (1,68 mmol) und 3,3 ml 1-Methyl-2-pyrrolidon werden ü. N. bei 50°C gerührt, dann abkühlen lassen. Die Mischung wird mit Wasser und Essigester versetzt. Die organische Phase wird getrennt, über Na₂SO₄ getrocknet und eingeengt. Man trocknet im Hochvakuum nach. Der Feststoff wird dann mit Cyclohexan/EE 2:1 versetzt, wobei Kristalle entstehen. Die Kristalle werden abgesaugt und mit Ether bei RT verrührt. Eine unlösliche Verunreinigung wird getrennt. Die etherische Lösung wird eingeengt und chromatographisch gereinigt. Man erhält 52,1 mg (18 %) 1-(2-Fluorbenzyl)-3-(4-methyl-3-hydroxymethyl-oxazol-2-yl)-pyrazolo[3,4b]pyridin. M.p. 145°C. R_{f}: 0,074 (Cyclohexan:EE 2:1). MS (ESI-POSITIV): 339 (100, [M + H]⁺).

### Beispiel 69

### 1-(2-Fluorbenzyl)-3-(4-ethyl-3-hydroxymethyl-oxazol-2-yl)-pyrazolo[3,4b]pyridin

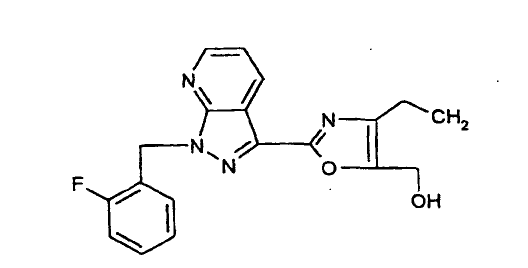

Die Verbindung wurde in Analogie zur Vorschrift des Beispiels 68 hergestellt. Ausbeute (52 % d. Th.), R_{f} 0,33 (Hexan:EE 1:1).

## Patentansprüche

1. Substituierte Pyrazolderivate der allgemeinen Formel (I), 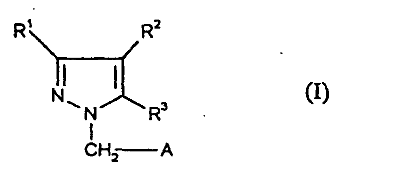 in welcher
R¹ für Imidazolyl, Pyridyl, Pyrimidyl oder , Oxazolyl steht, die gegebenenfalls bis zu 3-fach gleich oder verschieden durch Formyl, Fluor, Chlor, Amino, Mercaptyl, Cyano geradkettiges oder verzweigtes Acyl, Alkylthio, Alkoxy oder Alkoxycarbonyl mit jeweils bis zu 4 Kohlenstoffatomen oder geradkettiges oder verzweigtes Alkyl mit bis zu 4 Kohlenstoffatomen substituiert sind, das seinerseits durch Hydroxy, Carboxyl, Amino, Azido, geradkettiges oder verzweigtes Acyl, Alkoxy, Alkoxycarbonyl oder Acylamino mit jeweils bis zu 3 Kohlenstoffatomen substituiert sein kann,
und/oder durch einen Rest der Formel 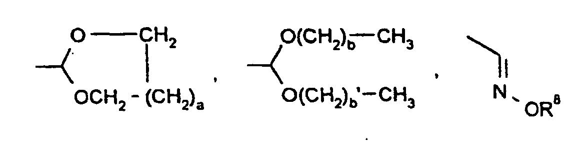 oder
-S(O)_{c}-NR⁹R¹⁰ substituiert sind,
worin
a, b und b' gleich oder verschieden sind und eine Zahl 0, 1 oder 2 bedeuten,
R⁸ Wasserstoff oder Methyl bedeutet,
c eine Zahl 1 oder 2 bedeutet und
R⁹ und R¹⁰ gleich oder verschieden sind und Wasserstoff oder geradkettiges oder verzweigtes Alkyl mit bis zu 9 Kohlenstoffatomen bedeuten, das gegebenenfalls durch Phenyl oder Naphthyl substituiert sein können, oder Phenyl oder Naphthyl bedeuten, die gegebenenfalls durch Fluor oder Chlor substituiert sind, oder Cyclopropyl oder Cycloheptyl bedeuten, oder
R⁹ und R¹⁰ gemeinsam mit dem Stickstoffatom einen Morpholinring oder einen Rest der Formel 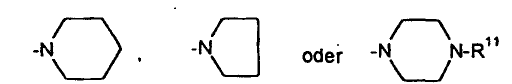 bilden,
worin
R¹¹ Wasserstoff, Methyl oder einen Rest der Formel 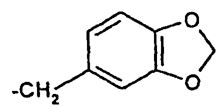 bedeutet oder Benzyl oder
Phenyl bedeutet, wobei die Ringsysteme gegebenenfalls durch Chlor substituiert sind,
R² und R³ unter Einbezug der Doppelbindung einen Pyridyl-, Pyrazinyl-Pyrimidinyl- oder Pyridazinylring bilden, die gegebenenfalls bis zu 3-fach gleich oder verschieden durch Formyl, Mercaptyl, Carboxyl, Hydroxy, geradkettiges oder verzweigtes Acyl, Alkoxy, Alkylthio oder Alkoxycarbonyl mit jeweils bis zu 4 Kohlenstoffatomen, Nitro, Cyano, Fluor, Chlor oder geradkettiges oder verzweigtes Alkyl oder Alkoxy mit jeweils bis zu 3 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 3 Kohlenstoffatomen substituiert sein kann,
und/oder die heterocyclischen Ringe gegebenenfalls durch Amino, N,N-Dimethylamino oder durch einen Rest der Formel -NH-CHO oder -N=CH-N(CH₃)₂ substituiert sind und/oder durch Phenyl substituiert sind, das seinerseits durch einen Rest der Formel -O(CH₂)₂-CH₃ substituiert sein kann,
A für Tetrahydropyranyl, Phenyl, Pyrimidyl, Thienyl oder Pyridyl steht, die gegebenenfalls bis zu 2-fach gleich oder verschieden durch Formyl, Carboxyl, geradkettiges oder verzweigtes Acyl, Alkylthio, Alkyloxyacyl, Alkoxy oder Alkoxycarbonyl mit jeweils bis zu 3 Kohlenstoffatomen, Fluor, Chlor, Brom, Nitro, Cyano, Trifluormethyl, oder geradkettiges oder verzweigtes Alkyl mit bis zu 3 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 3 Kohlenstoffatomen substituiert sein kann,
und deren isomere Formen und Salze.

2. Verfahren zur Herstellung von Verbindungen der allgemeinen Formel (I) gemäß Anspruch 1, **dadurch gekennzeichnet, daß** man in Abhängigkeit der verschiedenen Bedeutungen der unter R² und R³ definierten Heterocyclen entweder
[A] Verbindungen der allgemeinen Formel (II)
R¹-D (II)
in welcher
R¹ die oben angegebene Bedeutung hat,
und
D für Reste der Formel 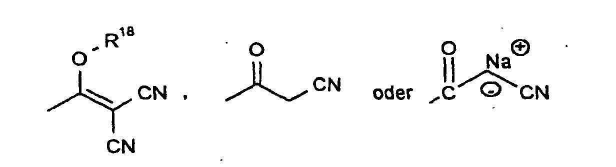 steht, in welchen
R¹⁸ für C₁-C₄-Alkyl steht,
durch Umsetzung mit Verbindungen der allgemeinen Formel (III)
A-CH₂-NH-NH₂ (III)
in welcher
A die oben angegebene Bedeutung hat
in inerten Lösemitteln, gegebenenfalls in Anwesenheit einer Base, in die Verbindungen der allgemeinen Formel (IV) oder (IVa) 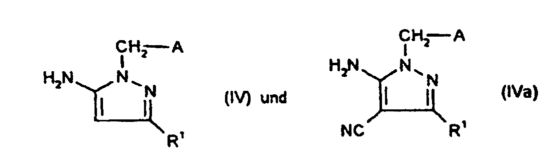 in welcher
A und R¹ die oben angegebene Bedeutung haben,
überführt,
und im Fall der Verbindungen der allgemeinen Formel (IVa) anschließend cyclisiert, mit Carbonsäuren, Nitrilen, Formamiden oder Guanidinosalzen
und im Fall der Verbindungen der allgemeinen Formel (IV) mit 1,3-Dicarbonyl-Derivaten, deren Salze, Tautomeren, Enolether oder Enaminen, in Anwesenheit von Säuren und gegebenenfalls unter Mikrowellen cyclisiert,
oder
[B] im Fall, daß R² und R³ gemeinsam einen Pyrazinring bilden, Verbindungen der allgemeinen Formel (IV) zunächst durch Nitrosierung in die Verbindungen der allgemeinen Formel (V) 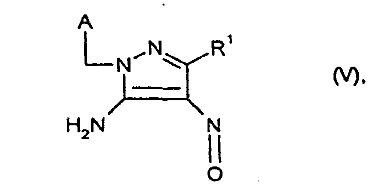 in welcher
A und R¹ die oben angegebene Bedeutung haben,
überführt,
in einem zweiten Schritt durch eine Reduktion die Verbindungen der allgemeinen Formel (VI) 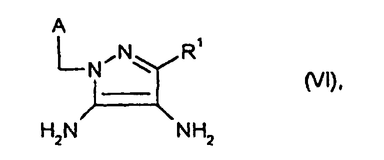 in welcher
A und R¹ die oben angegebene Bedeutung haben,
herstellt,
und abschließend mit 1,2-Dicarbonylverbindungen, vorzugsweise wäßriger Glyoxallösung cyclisiert,
oder
[C] Verbindungen der allgemeinen Formel (VII) 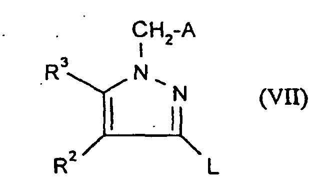 in welcher
A¹, R² und R³ die oben angegebene Bedeutung haben,
und
L für einen Rest der Formel -SnR¹⁹R²⁰R²¹, ZnR²², Iod, Brom oder Triflat steht,
worin
R¹⁹, R²⁰ und R²¹ gleich oder verschieden sind und geradkettiges oder verzweigtes Alkyl mit bis zu 4 Kohlenstoffatomen bedeuten,
und
R²² Halogen bedeutet,
mit Verbindungen der allgemeinen Formel (VIII)
R¹-T (VIII)
in welcher
R¹ die oben angegebene Bedeutung hat im
und im Fall L = SnR¹⁹R²⁰R²¹ oder ZnR²²
T für Triflat oder für Halogen, vorzugsweise für Brom steht,
und
im Fall L = Jod, Brom oder Triflat
T für einen Rest der Formel SnR^{19'}R^{20'}R^{21'}, ZnR^{22'} oder BR^{23'}R^{24'} steht,
worin
R^{19'}, R^{20'}, R^{21'} und R^{22'} die oben angebene Bedeutung von R¹⁹, R²⁰, R²¹ und R²² haben und mit dieser gleich oder verschieden sind,
R^{23'} und R^{24'} gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt, gegebenenfalls in Gegenwart einer Base,
[D] im Fall R¹ = 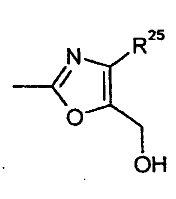 worin
R²⁵ (C₁-C₄)-Alkyl bedeutet,
Verbindungen der allgemeinen Formel (IX) 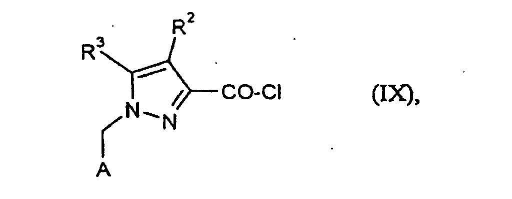 in welcher
A, R² und R³ die oben angegebene Bedeutung haben,
entweder direkt durch Umsetzung mit der Verbindung der Forme (X) 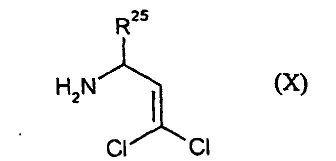 in welcher
R²⁵ die oben angegebene Bedeutung hat,
in dem System NaOCO-CH₃/N-Methylpyrrolidin in die Verbindungen der allgemeinen Formel (Ia) 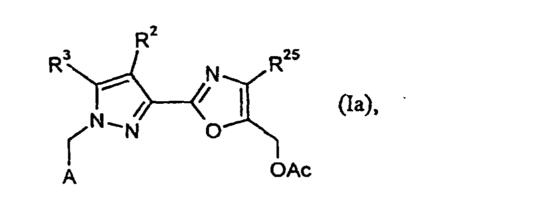 in welcher
R², R³ und A und R²⁵ die oben angegebene Bedeutung haben,
überführt,
und anschließend durch Einwirkung von Kaliumhydroxid in Methanol die Acetylgruppe abspaltet,
oder
zunächst durch Umsetzung der Verbindungen der allgemeinen Formel (IX) mit der Verbindung der Formel (X) die Verbindungen der allgemeinen Formel (XI) 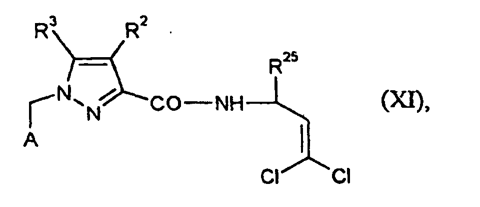 in welcher
R², R³, A und R²⁵ die oben angegebene Bedeutung haben,
herstellt,
und in einem weiteren Schritt durch Einwirkung von Kaliumhydroxid die Hydroxymethylverbindungen herstellt,
und gegebenenfalls durch eine Alkylierung nach üblichen Methoden in die entsprechende Alkoxyverbindungen überführt,
und im Fall der Gruppen -S(O)_{c}NR⁹R¹⁰ und -S(O)_{c'}NR^{9'}R^{10'} ausgehend von den unsubstituierten Verbindungen der allgemeinen Formel (I) zunächst mit Thionylchlorid und in einem zweiten Schritt mit den entsprechenden Aminen umsetzt
und gegebenenfalls die unter R¹, R², R³ und/oder A aufgeführten Substituenten nach üblichen Methoden, vorzugsweise durch Chlorierung, katalytische Hydrierung, Reduktion, Oxidation, Abspaltung von Schutzgruppen und/oder nucleophiler Substitution variiert oder einführt.

3. Arzneimittel enthaltend mindestens eine Verbindung der allgemeinen Formel (I) gemäß Anspruch 1.

4. Verfahren zur Herstellung von Arzneimitteln **dadurch gekennzeichnet, daß** man mindestens eine Verbindung der Formel (I) gemäß Anspruch 1, gegebenenfalls mit üblichen Hilfs- und Zusatzstoffen in eine geeignete Applikationsform überführt.

5. Arzneimittel enthaltend mindestens eine Verbindung der allgemeinen Formel (I) gemäß Anspruch 1 in Kombination mit organischen Nitraten oder NO-Donatoren.

6. Arzneimittel enthaltend mindestens eine Verbindung der allgemeinen Formel (I) gemäß Anspruch 1 in Kombination mit Verbindungen, die den Abbau von cyclischen Guanosinmonophosphat (cGMP) inhibieren.

7. Verwendung von Verbindungen der allgemeinen Formel (I) gemäß Anspruch 1 bei der Herstellung von Arzneimitteln zur Behandlung von Herz-Kreislauf-Erkrankungen.

8. Verwendung von Verbindungen der allgemeinen Formel (I) gemäß Anspruch 1 bei der Herstellung von Arzneimitteln zur Behandlung von thromboembolischen Erkrankungen und Ischämien.

## Claims

1. Substituted pyrazole derivatives of the general formula (I), 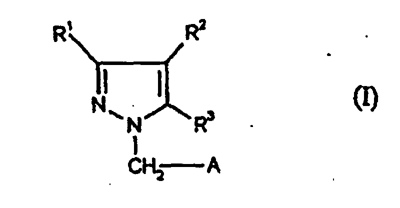 in which
R¹ represents imidazolyl, pyridyl, pyrimidyl or oxazolyl, each of which is optionally substituted up to 3 times identically or differently by formyl, fluorine, chlorine, amino, mercaptyl, cyano, straight-chain or branched acyl, alkylthio, alkoxy or alkoxycarbonyl in each case having up to 4 carbon atoms or straight-chain or branched alkyl having up to 4 carbon atoms, which for its part can be substituted by hydroxyl, carboxyl, amino, azido, straight-chain or branched acyl, alkoxy, alkoxycarbonyl or acylamino in each case having up to 3 carbon atoms,
and/or are substituted by a radical of the formula 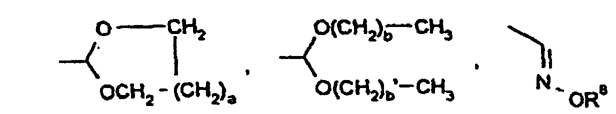 or
-S(O)_{c}-NR⁹R¹⁰,
in which
a, b and b' are identical or different and denote a number 0, 1 or 2,
R⁸ denotes hydrogen or methyl,
c denotes a number 1 or 2 and
R⁹ and R¹⁰ are identical or different and denote hydrogen or straight-chain or branched alkyl having up to 9 carbon atoms, which can optionally be substituted by phenyl or naphthyl, or
denote phenyl or naphthyl, each of which is optionally substituted by fluorine or chlorine, or
denote cyclopropyl or cycloheptyl, or
R⁹ and R¹⁰, together with the nitrogen atom, form a morpholine ring or a radical of the formula 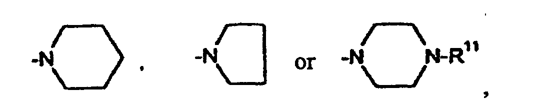 in which
R¹¹ denotes hydrogen, methyl or a radical of the formula 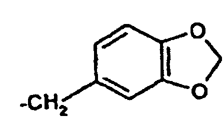 or denotes benzyl or phenyl, where the ring systems are optionally substituted by chlorine,
R² and R³, including the double bond, form a pyridyl, pyrazinyl, pyrimidinyl or pyridazinyl ring, each of which is optionally substituted up to 3 times identically or differently by formyl, mercaptyl, carboxyl, hydroxyl, straight-chain or branched acyl, alkoxy, alkylthio or alkoxycarbonyl in each case having up to 4 carbon atoms, nitro, cyano, fluorine, chlorine or straight-chain or branched alkyl or alkoxy in each case having up to 3 carbon atoms, which for its part can be substituted by hydroxyl, amino, carboxyl, straight-chain or branched acyl, alkoxy or alkoxycarbonyl in each case having up to 3 carbon atoms,
and/or the heterocyclic rings are optionally substituted by amino, N,N-dimethylamino or by a radical of the formula -NH-CHO or - N=CH-N(CH₃)₂ and/or by phenyl, which for its part can be substituted by a radical of the formula -O(CH₂)₂-CH₃,
**A** represents tetrahydropyranyl, phenyl, pyrimidyl, thienyl or pyridyl, each of which is optionally substituted up to 2 times identically or differently by formyl, carboxyl, straight-chain or branched acyl, alkylthio, alkyloxyacyl, alkoxy or alkoxycarbonyl in each case having up to 3 carbon atoms, fluorine, chlorine, bromine, nitro, cyano, trifluoromethyl, or straight-chain or branched alkyl having up to 3 carbon atoms, which for its part can be substituted by hydroxyl, carboxyl, straight-chain or branched acyl, alkoxy or alkoxycarbonyl in each case having up to 3 carbon atoms,
and their isomeric forms and salts.

2. Process for the preparation of compounds of the general formula (I) according to Claim 1, **characterized in that**, depending on the various meanings of the heterocycles defined under R² and R³, either
[A] compounds of the general formula (II)
R¹-D (II)
in which
R¹ has the meaning indicated above,
and
D represents radicals of the formula 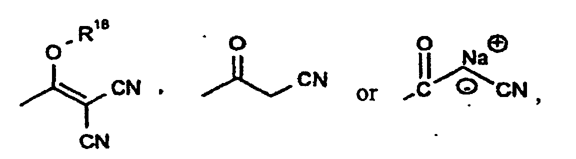
in which
R¹⁸ represents C₁-C₄-alkyl,
are converted by reaction with compounds of the general formula (III)
A-CH₂-NH-NH₂ (III)
in which
A has the meaning indicated above
in inert solvents, if appropriate in the presence of a base, into the compounds of the general formula (IV) or (IVa) 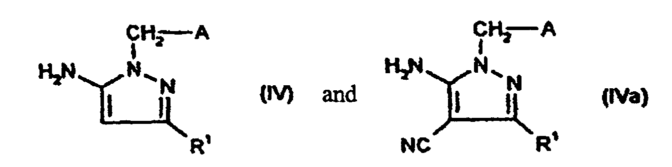 in which
A and R¹ have the meaning indicated above,
and, in the case of the compounds of the general formula (IVa), then cyclized with carboxylic acids, nitriles, formamides or guanidino salts
and, in the case of the compounds of the general formula (IV), cyclized with 1,3-dicarbonyl derivatives, their salts, tautomers, enol ethers or enamines, in the presence of acids and if appropriate under microwaves,
or
[B] in the case that R² and R³ together form a pyrazine ring, compounds of the general formula (IV) are first converted by nitrosation into the compounds of the general formula (V) 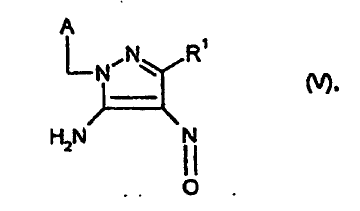 in which
A and R¹ have the meaning indicated above,
in a second step by means of a reduction the compounds of the general formula (VI) 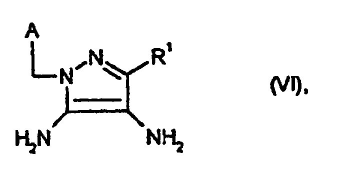 in which
A and R¹ have the meaning indicated above,
are prepared,
and finally cyclized with 1,2-dicarbonyl compounds, preferably aqueous glyoxal solution,
or
[C] compounds of the general formula (VII) 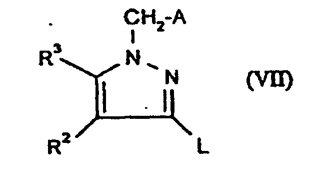 in which
A¹, R² and R₃ have the meaning indicated above,
and
L represents a radical of the formula -SnR¹⁹R²⁰R²¹, ZnR²², iodine, bromine or triflate,
in which
R¹⁹, R²⁰ and R²¹ are identical or different and denote straight-chain or branched alkyl having up to 4 carbon atoms,
and
R²² denotes halogen,
are reacted with compounds of the general formula (VIII)
R¹-T (VIII)
in which
R¹ has the meaning indicated above
and
if L = SnR¹⁹R²⁰R²¹ or ZnR²²
T represents triflate or halogen, preferably bromine,
and
if L = iodine, bromine or triflate
T represents a radical of the formula SnR^{19'}R^{20'}R^{21'}, ZnR^{22'} or BR^{23'}R^{24'},
in which
R^{19'}, R^{20'}, R^{21'} and R^{22'} have the meaning of R¹⁹, R²⁰, R²¹ and R²² indicated above and are identical to or different from this,
R^{23'} and R^{24'} are identical or different and denote hydroxyl, aryloxy having 6 to 10 carbon atoms or straight-chain or branched alkyl or alkoxy in each case having up to 5 carbon atoms, or together form a 5- or 6-membered carbocyclic ring,
in a palladium-catalysed reaction in inert solvents, if appropriate in the presence of a base,
[D] if R¹ = 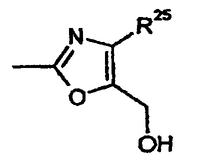 in which
R²⁵ denotes (C₁-C₄)-alkyl,
compounds of the general formula (IX) 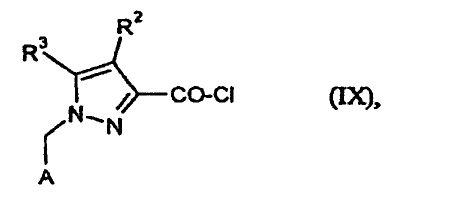 in which
A, R² and R³ have the meaning indicated above,
are converted, either directly by reaction with the compound of the formula (X) 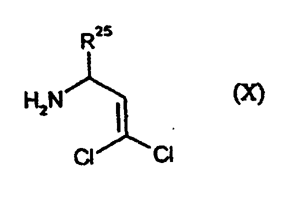 in which
R²⁵ has the meaning indicated above,
in the system NaOCO-CH₃/N-methylpyrrolidine
into the compounds of the general formula (Ia) 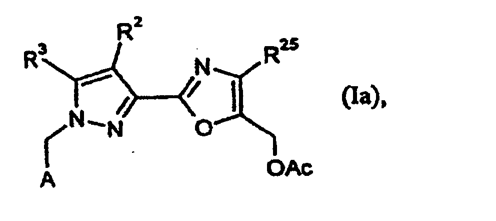 in which
R², R³, A and R²⁵ have the meaning indicated above,
and then the acetyl group is removed by the action of potassium hydroxide in methanol,
or
first, by reaction of the compounds of the general formula (IX) with the compound of the formula (X), the compounds of the general formula (XI) 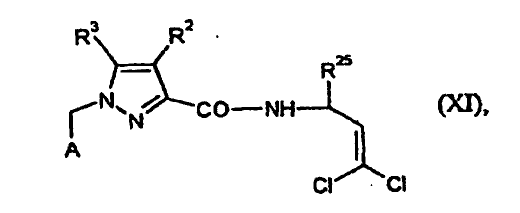 in which
R², R³, A and R²⁵ have the meaning indicated above,
are prepared,
and in a further step the hydroxymethyl compounds are prepared by the action of potassium hydroxide,
and, if appropriate, converted into the corresponding alkoxy compounds by an alkylation according to customary methods,
and in the case of the groups -S(O)_{c}-NR⁹R¹⁰ and -S(O)_{c'}-NR^{9'}R^{10'}, starting from the unsubstituted compounds of the general formula (I), first reacted with thionyl chloride and in a second step reacted with the corresponding amines
and, if appropriate, the substituents mentioned under R¹, R², R³ and/or A are varied or introduced according to customary methods, preferably by chlorination, catalytic hydrogenation, reduction, oxidation, removal of protective groups and/or nucleophilic substitution.

3. Medicaments comprising at least one compound of the general formula (I) according to Claim 1.

4. Process for the production of medicaments, **characterized in that** at least one compound of the formula (I) according to Claim 1 is converted into a suitable administration form, if appropriate using customary excipients and additives.

5. Medicaments comprising at least one compound of the general formula (I) according to Claim 1 in combination with organic nitrates or NO donors.

6. Medicaments comprising at least one compound of the general formula (I) according to Claim 1 in combination with compounds which inhibit the degradation of cyclic guanosine monophosphate (cGMP).

7. Use of compounds of the general formula (I) according to Claim 1 in the production of medicaments for the treatment of cardiovascular diseases.

8. Use of compounds of the general formula (I) according to Claim 1 in the production of medicaments for the treatment of thromboembolic diseases and ischaemias.

## Revendications

1. Dérivés de pyrazole substitués de formule générale (I) 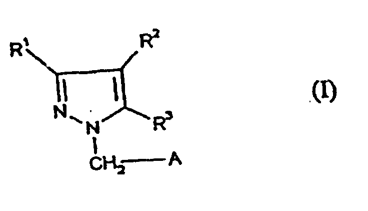 dans laquelle
R¹ est un reste imidazolyle, pyridyle, pyrimidyle ou oxazolyle, ces restes étant éventuellement substitués jusqu'à 3 fois identiques ou différents par un radical formyle, fluoro, chloro, amino, mercaptyle, cyano, un radical acyle, alkylthio, alkoxy ou alkoxycarbonyle linéaire ou ramifié, ayant chacun jusqu'à 4 atomes de carbone ou un radical alkyle linéaire ou ramifié ayant jusqu'à 4 atomes de carbone, qui peut lui-même être substitué par un radical hydroxy, carboxyle, amino, azido, un radical acyle, alkoxy, alkoxycarbonyle ou acylamino linéaire ou ramifié ayant chacun jusqu'à 3 atomes de carbone, et/ou par un reste de formule 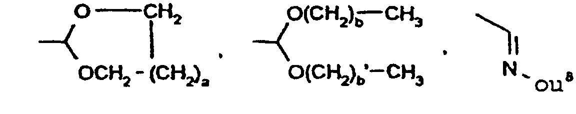 ou **-S(O)**_{**c**}**-NR**^{**9**}**R**^{**10**}
dans laquelle
a, b et b' sont égaux ou différents et représentent le nombre 0, 1 ou 2, ,
R⁸ représente l'hydrogène ou le groupe méthyle,
c est le nombre 1 ou 2, R⁹ et R¹⁰ sont identiques ou différents et représentent l'hydrogène ou un reste alkyle linéaire ou ramifié ayant jusqu'à 9 atomes de carbone, qui peut éventuellement être substitué par un radical phényle ou naphtyle, ou représentent
un reste phényle ou un reste naphtyle, qui sont éventuellement substitués par du fluor ou du chlore, ou représentent
un reste cyclopropyle ou cycloheptyle ou bien
R⁹ et R¹⁰ forment conjointement avec l'atome d'azote, un noyau de morpholine ou un reste de formule 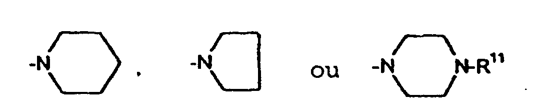 dans laquelle
R¹¹ désigne l'hydrogène, le reste méthyle ou un reste de formule 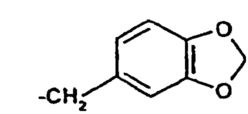 ou représente un reste benzyle
ou phényle, les systèmes cycliques étant éventuellement substitués par du chlore,
R² et R³ y compris la double liaison forment un noyau pyridyle, pyrazinyle, pyrimidinyle ou pyridazinyle, ces noyaux étant éventuellement substitués jusqu'à 3 fois identiques ou différents par un radical formyle, mercaptyle, carboxyle, hydroxy, un radical acyle, alkoxy, alkylthio, ou alkoxycarbonyle linéaire ou ramifié ayant chacun jusqu'à 4 atomes de carbone, un radical nitro, cyano, fluoro, chloro ou un radical alkyle ou alkoxy linéaire ou ramifié ayant chacun jusqu'à 3 atomes de carbone, pouvant lui-même porter un radical hydroxy, amino, carboxyle, un radical acyle, alkoxy ou alkoxycarbonyle linéaire ou ramifié ayant jusqu'à 3 atomes de carbone,
et/ou les noyaux hétérocycliques sont éventuellement substitués par un radical amino, N,N-diméthylamino ou par un reste de formule -NH-CHO ou -N=CH-N(CH₃)₂ et/ou par un reste phényle qui peut lui-même être substitué par un reste de formule -O(CH₂)₂₋ CH₃,
A est un reste tétrahydropyrannyle, phényle, pyrimidyle, thiényle ou pyridyle, pouvant porter éventuellement jusqu'à 2 substituants, identiques ou différents, formyle, carboxyle ou acyle, alkylthio, alkyloxyacyle, alkoxy ou alkoxycarbonyle linéaire ou ramifié ayant chacun jusqu'à 3 atomes de carbone, fluor, chlore, brome, nitro, cyano, trifluorméthyle, ou bien alkyle linéaire ou ramifié ayant jusqu'à 3 atomes de carbone qui peut lui-même être substitué par un radical hydroxy, carboxyle, un radical acyle, alkoxy ou alkoxycarbonyle linéaire ou ramifié ayant chacun jusqu'à 3 atomes de carbone,
et leurs formes isomères et leurs sels.

2. Procédé de production de composés de formule générale (I) suivant la revendication 1, **caractérisé en ce que**, en fonction des diverses significations des hétérocycles définies pour R² et R³,
[A] on transforme des composés de formule générale (II)
R¹-D (II)
dans laquelle
R¹ a la définition indiquée ci-dessus,
et
D représente des restes de formule 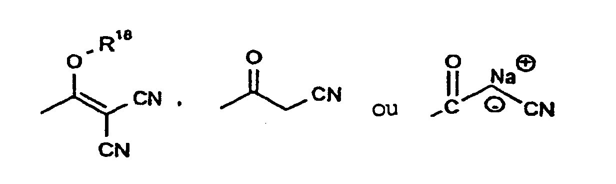
dans lesquels
R¹⁸ est un reste alkyle en C₁ à C₄,
par réaction avec des composés de formule générale (III)
A-CH₂-NH-NH₂ (III)
dans laquelle
A a la définition indiquée ci-dessus
dans des solvants inertes, éventuellement en présence d'une base, en les composés de formule générale (IV) ou (IVa) 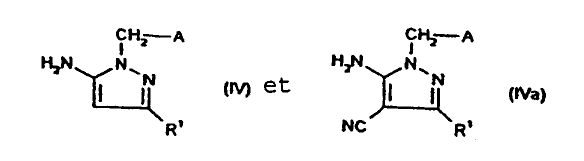 dans laquelle
A et R¹ ont la définition indiquée ci-dessus,
et dans le cas des composés de formule générale (IVa), on effectue ensuite une cyclisation, avec des acides carboxyliques, des nitriles, des formamides ou des sels de guanidine,
et dans le cas des composés de formule générale (IV), on effectue une cyclisation avec des dérivés 1,3-dicarbonyliques, leurs sels, leurs tautomères, leurs éthers d'énol ou leurs énamines, en présence d'acides et le cas échéant, sous l'action de micro-ondes,
ou bien
[B] au cas où R² et R³ forment ensemble un noyau de pyrazine, on transforme tout d'abord des composés de formule générale (IV) par nitrosation en composés de formule générale (V) 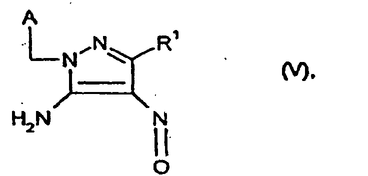 dans laquelle
A et R¹ ont la définition indiquée ci-dessus dans une deuxième étape, on prépare par réduction les composés de formule générale (VI) 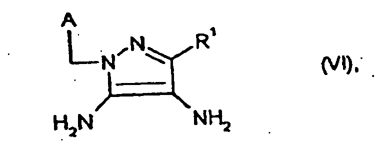 dans laquelle
A et R¹ ont la définition indiquée ci-dessus,
et finalement, on effectue une cyclisation avec des composés 1,2-dicarbonyliques, de préférence une solution aqueuse de glyoxal
ou bien
[C] on fait réagir des composés de formule générale (VII) 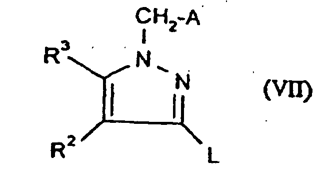 dans laquelle
A¹, R² et R³ ont la définition indiquée ci-dessus
et
L représente un reste de formule - SnR¹⁹R²⁰R²¹, ZnR²², l'iode, le brome ou un reste triflat,
où
R¹⁹, R²⁰ et R²¹ sont identiques ou différents et désignent un resté alkyle linéaire ou ramifié ayant jusqu'à 4 atomes de carbone,
et
R²² représente un halogène,
avec des composés de formule générale (VIII)
R¹-T (VIII)
dans laquelle
R¹ a la définition indiquée ci-dessus
et
au cas où L représente SnR¹⁹R²⁰R²¹ ou ZnR²²
T est un reste triflat ou un halogène, de préférence le brome,
et
au cas où L représente l'iode, le brome ou le reste triflat
T est un reste de formule SnR^{19'}R^{20'}R^{21'}, ZnR^{22'} ou BR^{23'}R^{24'}
où
R^{19'}, R^{20'}, R^{21'} et R^{22'} ont la définition indiquée ci-dessus pour R¹⁹, R²⁰, R²¹ et R²² et y sont identiques ou en sont différents,
R^{23'} et R^{24'}sont identiques ou différents et désignent un groupe hydroxy, un reste aryloxy ayant 6 à 10 atomes de carbone ou un reste alkyle ou alkoxy linéaire ou ramifié ayant chacun jusqu'à 5 atomes de carbone, ou forment ensemble un noyau carbocyclique pentagonal ou hexagonal,
dans une réaction catalysée par le palladium dans des solvants inertes, éventuellement en présence d'une base,
[D] au cas où R¹ représente le groupe 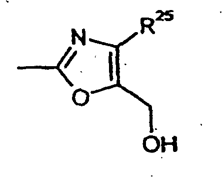
dans lequel
R²⁵ est un reste alkyle en C₁ à C₄,
on transforme des composés de formule générale (IX) 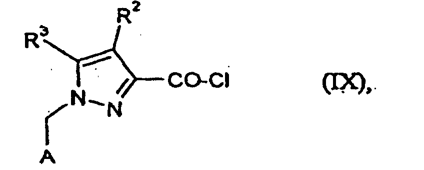 dans laquelle
A, R² et R³ ont la définition indiquée ci-dessus, soit directement par réaction avec le composé de formule (X) 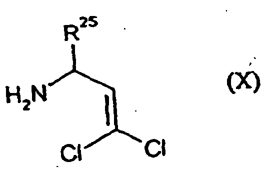 dans laquelle
R²⁵ a la définition indiquée ci-dessus,
dans le système NaOCO-CH₃/N-méthyl-pyrrolidine en composés de formule générale. (Ia) 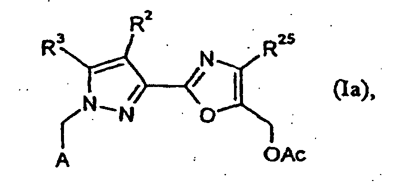 dans laquelle
R², R³ et A et R²⁵ ont la définition indiquée ci-dessus, puis on élimine le groupe acétyle par l'action de l'hydroxyde de potassium dans du méthanol,
soit
on prépare tout d'abord par réaction de composés de formule générale (IX) avec le composé de formule (X) les composés de formule générale (XI) 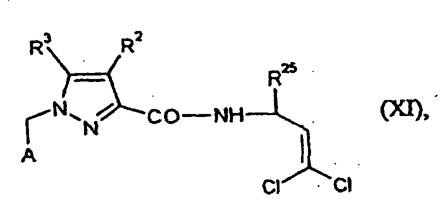 dans laquelle
R², R³, A et R²⁵ ont la définition indiquée ci-dessus,
et on prépare les composés hydroxyméthyliques dans une autre étape par l'action de l'hydroxyde de potassium,
et on les transforme, le cas échéant, par alkylation selon des modes opératoires classiques en les composés alkoxylés correspondants
et dans le cas des groupes -S(CO)_{c}NR⁹R¹⁰ et - S(CO)_{c'}NR^{9'}R^{10'}, en partant des composés non substitués de formule générale (I), on les fait réagir tout d'abord avec le chlorure de thionyle et, dans une seconde étape, avec les amines correspondantes
et, le cas échéant, on fait varier ou on introduit les substituants énumérés pour R¹, R², R³ et/ou A selon des modes opératoires classiques, de préférence par chloration, hydrogénation catalytique, réduction, oxydation, élimination de groupes protecteurs et/ou substitution nucléophile.

3. Médicament contenant au moins un composé de formule générale (I) suivant la revendication 1.

4. Procédé de préparation de médicaments, **caractérisé en ce qu'**on fait prendre une forme d'administration convenable à au moins un composé de formule (I) suivant la revendication 1, éventuellement avec des substances auxiliaires et des additifs classiques.

5. Médicament contenant au moins un composé de formule générale (I) suivant la revendication 1, en association avec des nitrates organiques ou des donneurs organiques de NO.

6. Médicament contenant au moins un composé de formule générale (I) suivant la revendication 1, en association avec des composés qui inhibent la dégradation du monophosphate cyclique de guanosine (cGMP).

7. Utilisation de composés de formule générale (I) suivant la revendication 1 dans la préparation de médicaments destinés au traitement de maladies du système circulatoire.

8. Utilisation de composés de formule générale (I) suivant la revendication 1 pour la préparation de médicaments destinés au traitement de maladies thromboemboliques et d'ischémies.
